(19) Europäisches Patentamt
European Patent Office
Office européen des brevets

(11) EP 1 960 007 B1

(12) EUROPEAN PATENT SPECIFICATION

(45) Date of publication and mention
of the grant of the patent:
06.10.2010 Bulletin 2010/40

(51) Int Cl.:
*A61L 15/48* (2006.01)    *A61L 15/60* (2006.01)
*A61L 15/22* (2006.01)

(21) Application number: 06838812.3

(22) Date of filing: 30.11.2006

(86) International application number:
PCT/US2006/046051

(87) International publication number:
WO 2007/070262 (21.06.2007 Gazette 2007/25)

(54) **ABSORBENT ARTICLES COMPRISING THERMOPLASTIC COATED SUPERABSORBENT POLYMER MATERIALS**

SAUGFÄHIGE ARTIKEL MIT WÄRMEKUNSTSTOFFBESCHICHTETEN SUPERSAUGFÄHIGEN POLYMERMATERIALIEN

ARTICLES ABSORBANTS COMPRENANT DES MATERIAUX POLYMERIQUES SUPERABSORBANTS ENDUITS DE MATERIAUX THERMOPLASTIQUES

(84) Designated Contracting States:
DE GB

(30) Priority: 12.12.2005 US 302031

(43) Date of publication of application:
27.08.2008 Bulletin 2008/35

(73) Proprietor: KIMBERLY-CLARK WORLDWIDE, INC.
Neenah, WI 54956 (US)

(72) Inventors:
• QIN, Jian
Appleton, WI 54915 (US)
• SMITH, Scott, J.
Greensboro, NC 27407 (US)
• MCINTOSH, Stan
Greensboro, NC 27407 (US)
• JONES LANG, Angela
High Point, NC 27265 (US)
• BERGMAN, David, L., Jr.
Greensboro, NC 27410 (US)

(74) Representative: Zimmermann & Partner
Postfach 330 920
80069 München (DE)

(56) References cited:
WO-A-2006/007182    WO-A-2007/070776
US-A1- 2005 027 268    US-A1- 2005 096 435

**Description**

**BACKGROUND**

Absorbent articles are useful for absorbing many types of fluids, including fluids secreted or eliminated by the human body. Superabsorbent materials (SAM's) are frequently used in absorbent articles to help improve the absorbent properties of such articles. Superabsorbent materials are generally polymer based and are available in many forms, such as powders, granules, microparticles, films and fibers, for example. Upon contact with fluids, such superabsorbent materials swell by absorbing the fluids into their structures. In general, superabsorbent materials can quickly absorb fluids insulted into such articles, and can retain such fluids to prevent leakage and help provide a dry feel even after fluid insult.

There is a continuing effort to improve the performance of such absorbent articles, especially at high levels of fluid saturation, to thereby reduce the occurrence of leakage. This is particularly significant when such articles are subjected to repeated fluid insults during use. This has become an increasing challenge as recent efforts in absorbent article design have generally focused on using higher concentrations of superabsorbent material and less fluff fibers to make the absorbent structures thinner and denser. However, notwithstanding the increase in total absorbent capacity obtained by increasing the concentration of superabsorbent material, such absorbent articles may still nevertheless leak during use. Such leakage may in part be the result of the absorbent core having an insufficient intake rate (i.e., the rate at which a fluid insult can be taken into and entrained within the absorbent core for subsequent absorption by the superabsorbent material) due to lack of available void volume. Therefore, there is a desire for an absorbent article which contains high levels of superabsorbent materials and which maintains a sufficient intake rate.

In addition, there is also a need for superabsorbent polymer materials that have increased permeability characteristics as well as increased affinity to thermoplastic fibers, such as polyolefins for example, while retaining other characteristics such as adequate absorption and retention. Permeability is a measure of the effective connectedness of a porous structure, be it a mat of fiber or a slab of foam or, in this case, crosslinked polymers and may be specified in terms of the void fraction and extent of connectedness of the superabsorbent polymer material. Gel permeability is a property of the mass of particles as a whole and is related to particle size distribution, particle shape, and the connectedness of the open pores, shear modulus and surface modification of the swollen gel. In practical terms, the permeability of the superabsorbent polymer material is a measure of how rapidly liquid flows through the mass of swollen particles. Low permeability indicates that liquid cannot flow readily through the superabsorbent polymer material, which is generally referred to as gel blocking, and that any forced flow of liquid (such as a second application of urine during use of the diaper) must take an alternate path (e.g., diaper leakage). Therefore, there is a desire for an absorbent article which exhibits increased permeability characteristics.

To obtain a superabsorbent polymer material with high gel strength, the degree of crosslinking of the polymer may be increased, which necessarily results in a reduction in the swellability and the retention capacity. To achieve the increased permeabilities needed in extremely thin, next generation-type articles with low fiber content, current art has taught to increase the amount of crosslinking. However the absorption and retention values of the superabsorbent polymer material are reduced to undesirably low levels. It is an important goal of the art of making superabsorbent polymer material to develop a composition having a high absorption and retention capacity for liquid in the after-surface crosslinking stage and increased permeability properties. It has been found that by using new surface modifications to the superabsorbent polymer particles, results of higher permeabilities without the undesirable associated low absorption values are achieved.

Additionally, increasing the performance of one absorbent property of an absorbent article can often result in an adverse effect on other absorbent properties. For example, an increase in fluid intake rate can often result in a decrease in capacity. While an increase in fluid intake rate is generally desirable, a corresponding decrease in capacity is generally undesirable. Therefore, there is an additional desire for an absorbent article which exhibits an improved fluid intake rate without adversely affecting other absorbent properties, such as capacity. Furthermore, there is a desire to accomplish such an improvement without resorting to complex, capital-intensive absorbent fabrication processes or additional non-absorbent binder fiber components. Such means should be compatible with conventional, low cost, efficient air-forming equipment that is widely used in the industry and integrated in the absorbent article manufacturing process.

**SUMMARY**

In response to the needs discussed above, an absorbent core of the present invention comprises a substrate and a superabsorbent polymer material. The superabsorbent material comprises from 55 to 99.9% by weight of a polymerizable unsaturated acid group containing monomer, based on the weight of the superabsorbent polymer. The superabsorbent polymer material also comprises from 0.001 to 5% by weight of internal crosslinking agent, based on the weight of the polymerizable unsaturated acid group containing monomer. The polymerizable unsaturated acid group containing monomer and the internal crosslinking agent are polymerized and prepared into the superabsorbent polymer material.

The super absorbent polymer material is coated with from 0.001 to 5% by weight of surface crosslinking agent: from 0.01 to 10% by weight of a penetration modifier; from 0 to 5% by weight of a multivalent metal salt; from 0 to 2% by weight of surfactant; from 0.01 to 5% by weight of an insoluble, inorganic powder; and from 0.01 to 5% by weight of a thermoplastic polymer comprising maleated polypropylene (MPP), each based on the weight of the dry superabsorbent polymer material. In particular aspects, the surface treated superabsorbent polymer materials are heat-treated. In some aspects, the surface treated superabsorbent polymer materials have a thermoplastic melt temperature wherein the thermoplastic polymer is applied on the superabsorbent material surface coincident with or followed by a temperature of the coated superabsorbent polymer material of at least the thermoplastic melt temperature or greater. The superabsorbent polymer material is post-treated with a cationic polymer such that the superabsorbent particles comprise a coating of from 0.01 to 5 % by weight of a cationic polymer, based on the superabsorbent polymer material. In yet other aspects, the superabsorbent polymer material comprises particles in a size range of 300 to 600 microns, such as at least 50% of the particles are in a size range of 300 to 600 microns.

In some aspects, the superabsorbent polymer material has a centrifuge retention capacity of 23 g/g or more, as measured by the Centrifuge Retention Capacity Test. In other aspects, the superabsorbent polymer material has a free swell gel bed permeability of 100 Darcy or more, as measured by the Free Swell Gel Bed Permeability Test. In still other aspects, the superabsorbent polymer material has a superabsorbent material shake-out of less than 20%, as measured by the Oven Shake-Out Test.

In some aspects, the absorbent core further comprises a meltblown thermoplastic fiber and exhibits a Stretchability of at least 50% as measured by the Composite Stretchability Test, a Composite Permeability of at least 15 Darcy as measured by the Composite Permeability Test, at least one FIFE Intake Rate greater than 1 g/sec as measured by the Fluid Intake Rate Test, and a Composite Shakeout value of not more than 2% by weight as measured by the Composite Shakeout Test. The result is an absorbent composite which exhibits improved performance as well as greater comfort and confidence among the user.

Numerous other features and advantages of the present invention will appear from the following description. In the description, reference is made to exemplary embodiments of the invention. Such embodiments do not represent the full scope of the invention. Reference should therefore be made to the claims herein for interpreting the full scope of the invention.

## FIGURES

The foregoing and other features, aspects and advantages of the present invention will become better understood with regard to the following description, appended claims and accompanying drawings where:

FIG 1 is a perspective view of one embodiment of an absorbent article that may be made in accordance with the present invention;

FIG 2 is a plan view of the absorbent article shown in FIG 1 with the article in an unfastened, unfolded and laid flat condition showing the surface of the article that faces the wearer when worn and with portions cut away to show underlying features;

FIG 3 is a schematic diagram of one version of a method and apparatus for producing an absorbent core;

FIG 4 is a cross-sectional side view of a layered absorbent core according to the present invention;

FIG 5A is a cross-section side view of an absorbent bandage of the present invention;

FIG 5B is a top perspective view of an absorbent bandage of the present invention;

FIG 6 is a top perspective view of an absorbent bed or furniture liner of the present invention;

FIG 7 is a cross-section of an apparatus for conducting a Gel Bed Permeability Test;

FIG 8 is a section taken in the plane of line 2-2 of FIG 7;

FIG 9 is a partially cut away top view of a Saturated Capacity tester;

FIG 10 is a side view of a Saturated Capacity tester;

FIG 11 is a rear view of a Saturated Capacity tester;

FIG 12 is a top view of the test apparatus employed for the Fluid Intake Rate Test; and

FIG 13 is a side view of the test apparatus employed for the Fluid Intake Rate Test.

Repeated use of reference characters in the present specification and drawings is intended to represent the same or analogous features or elements of the present invention.

**DEFINITIONS**

It should be noted that, when employed in the present disclosure, the terms "comprise," "comprising" and other derivatives from the root term "comprise" are intended to be open-ended terms that specify the presence of any stated features, elements, integers, steps, or components, and are not intended to preclude the presence or addition of one or more other features, elements, integers, steps, components, or groups thereof.
The term "absorbent article" generally refers to devices which can absorb and contain fluids. For example, personal care absorbent articles refer to devices which are placed against or near the skin to absorb and contain the various fluids discharged from the body. The term "disposable" is used herein to describe absorbent articles that are not intended to be laundered or otherwise restored or reused as an absorbent article, after a single use. Examples of such disposable absorbent articles include, but are not limited to, personal care absorbent articles, health/medical absorbent articles, and household/industrial absorbent articles.
The term "coform" is intended to describe a blend of meltblown fibers and cellulose fibers that is formed by air forming a meltblown polymer material while simultaneously blowing air-suspended cellulose fibers into the stream of meltblown fibers. The coform material may also include other materials, such as superabsorbent materials. The meltblown fibers containing wood fibers are collected on a forming surface, such as provided by a foraminous belt. The forming surface may include a gas-pervious material, such as spunbonded fabric material, that has been placed onto the forming surface.
The term "Darcy" is a CGS unit of permeability. One Darcy is the permeability of a solid through which one cubic centimeter of fluid, having a viscosity of one centipoise, will flow in one second through a section one centimeter thick and one square centimeter in cross section, if the pressure difference between the two sides of the solid is one atmosphere. It turns out that permeability has the same units as area; since there is no SI unit of permeability, square meters are used. One Darcy is equal to about $0.98692 \times 10^{-12}$ square meter or about $0.98692 \times 10^{-8}$ square centimeter.
The term "dry superabsorbent polymer compostion" or "dry superabsorbent polymer material" generally refers to the superabsorbent polymer material having less than about 10% moisture.
The terms "elastic," "elastomeric" and "elastically extensible" are used interchangeably to refer to a material or composite that generally exhibits properties which approximate the properties of natural rubber. The elastomeric material is generally capable of being extended or otherwise deformed, and then recovering a significant portion of its shape after the extension or deforming force is removed.
The term "extensible" refers to a material that is generally capable of being extended or otherwise deformed, but which does not recover a significant portion of its shape after the extension or deforming force is removed.
The term "fluid impermeable," when used to describe a layer or laminate, means that fluid such as water or bodily fluids will not pass substantially through the layer or laminate under ordinary use conditions in a direction generally perpendicular to the plane of the layer or laminate at the point of fluid contact.
The term "health/medical absorbent article" includes a variety of professional and consumer health-care products including, but not limited to, products for applying hot or cold therapy, medical gowns (i.e., protective and/or surgical gowns), surgical drapes, caps, gloves, face masks, bandages, wound dressings, wipes, covers, containers, filters, disposable garments and bed pads, medical absorbent garments, underpads, and the like.
The term "househoid/industrial absorbent articles" include construction and packaging supplies, products for cleaning and disinfecting, wipes, covers, filters, towels, disposable cutting sheets, bath tissue, facial tissue, nonwoven roll goods, home-comfort products including pillows, pads, mats, cushions, masks and body care products such as products used to cleanse or treat the skin, laboratory coats, cover-alls, trash bags, stain removers, topical compositions, pet care absorbent liners, laundry soil/ink absorbers, detergent agglomerators, lipophilic fluid separators, and the like.
The terms "hydrophilic" and "wettable" are used interchangeably to refer to a material having a contact angle of water in air of less than 90 degrees. The term "hydrophobic" refers to a material having a contact angle of water in air of at least 90 degrees. For the purposes of this application, contact angle measurements are determined as set forth in Robert J. Good and Robert J. Stromberg, Ed., in "Surface and Colloid Science - Experimental Methods," Vol. II, (Plenum Press, 1979).

The term "insult target zone" refers to an area of an absorbent core where it is particularly desirable for the majority of a fluid insult, such as urine, menses, or bowel movement, to initially contact. In particular, for an absorbent core with one or more fluid insult points in use, the insult target zone refers to the area of the absorbent core extending a distance equal to 15% of the total length of the core from each insult point in both directions.

The term "materials" when used in the phrase "superabsorbent materials" refers generally to discrete units. The units can comprise particles, granules, fibers, flakes, agglomerates, rods, spheres, needles, particles coated with fibers or other additives, pulverized materials, powders, films, and the like, as well as combinations thereof. The materials can have any desired shape such as, for example, cubic, rod-like, polyhedral, spherical or semi-spherical, rounded or semi-rounded, angular, irregular, etc. Additionally, superabsorbent materials may be composed of more than one type of material.

The term "meltblown fibers" refers to fibers formed by extruding a molten thermoplastic material through a plurality of fine, usually circular, die capillaries as molten threads or filaments into a high velocity, usually heated, gas (e.g., air) stream which attenuates the filaments of molten thermoplastic material to reduce their diameter. Thereafter, the meltblown fibers are carried by the high velocity gas stream and are deposited on a collecting surface to form a web of randomly disbursed meltblown fibers.

The terms "nonwoven" and "nonwoven web" refer to materials and webs of material having a structure of individual fibers or filaments which are interlaid, but not in an identifiable manner as in a knitted fabric. The terms "fiber" and "filament" are used herein interchangeably. Nonwoven fabrics or webs have been formed from many processes such as, for example, meltblowing processes, spunbonding processes, air laying processes, and bonded-carded-web processes. The basis weight of nonwoven fabrics is usually expressed in ounces of material per square yard (osy) or grams per square meter (gsm) and the fiber diameters are usually expressed in microns. (Note that to convert from osy to gsm, multiply osy by 33.91.)

The terms "particle," "particles," "particulate," "particulates" and the like, when used with the term "superabsorbent" or "superabsorbent polymer" refers to the form of discrete units. The units can comprise flakes, fibers, agglomerates, granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "personal care absorbent article" includes, but is not limited to, absorbent articles such as diapers, diaper pants, baby wipes, training pants, absorbent underpants, child care pants, swimwear, and other disposable garments; feminine care products including sanitary napkins, wipes, menstrual pads, menstrual pants, panty liners, panty shields, interlabials, tampons, and tampon applicators; adult-care products including wipes, pads such as breast pads, containers, incontinence products, and urinary shields; clothing components; bibs; athletic and recreation products; and the like.

The term "polymers" includes, but is not limited to, homopolymers, copolymers, such as for example, block, graft, random and alternating copolymers, terpolymers, etc. and blends and modifications thereof. Furthermore, unless otherwise specifically limited, the term "polymer" shall include all possible configurational isomers of the material. These configurations include, but are not limited to isotactic, syndiotactic and atactic symmetries:

The term "polyolefin" as used herein generally includes, but is not limited to, materials such as polyethylene, polypropylene, polyisobutylene, polystyrene, ethylene vinyl acetate copolymer and the like, the homopolymers, copolymers, terpolymers, etc., thereof, and blends and modifications thereof. The term "polyolefin" shall include all possible structures thereof, which includes, but is not limited to, isotatic, synodiotactic and random symmetries. Copolymers include random and block copolymers.

The terms "spunbond" and "spunbonded fiber" refer to fibers which are formed by extruding filaments of molten thermoplastic material from a plurality of fine, usually circular, capillaries of a spinneret, and then rapidly reducing the diameter of the extruded filaments.

The term "stretchable" refers to materials which may be extensible or which may be elastically extensible.

The terms "superabsorbent" and "superabsorbent materials" refer to water swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In contrast, "absorbent material" are capable, under the most favorable conditions, of absorbing at least 5 times their weight of an aqueous solution containing 0.9 weight percent sodium chloride.

The term "thermoplastic" describes a material that softens when exposed to heat and which substantially returns to a non-softened condition when cooled to room temperature.

These terms may be defined with additional language in the remaining portions of the specification.

## DETAILED DESCRIPTION

An absorbent core of the present invention comprises a substrate and a superabsorbent polymer material. The superabsorbent maternal comprises from 55 to 99.9% by weight of a polymerizable unsaturated acid group containing monomer, based on the weight of the superabsorbent polymer. The superabsorbent polymer material also comprises from 0.001 to 5% by weight of an internal crosslinking agent, based on the weight of the polymerizable unsaturated acid group containing monomer. The polymerizable unsaturated acid group containing monomer and the internal crosslinking agent are polymerized and prepared into the superabsorbent polymer material.

The superabsorbent polymer material is coated with from 0.001 to 5% by weight of surface crosslinking agent; from 0.01 to 10% by weight of a penetration modifier; from 0 to 5% by weight of a multivalent metal salt; from 0 to 2% by weight of surfactant from 0.01 to 5% by weight of an insoluble, inorganic powder; and from 0.01 to 5% by weight of a thermoplastic polymer comprising maleated polypropylene (MPP), each based on the weight of the dry superabsorbent polymer material. In particular aspects, the surface treated superabsorbent polymer materials are heat-treated. In some aspects, the surface treated superabsorbent polymer materials have a thermoplastic melt temperature wherein the thermoplastic polymer is applied on the superabsorbent material surface coincident with or followed by a temperature of the coated superabsorbent polymer material of at least the thermoplastic melt temperature or greater. The superabsorbent polymer material is post treated with a cationic polymer such as to comprise a coating of from 0.01 to 5 % by weight of said cationic polymer. In yet other aspects, the superabsorbent polymer material comprises particles in a size range of 300 to 600 microns, such as at least 50% of the particles are in a size range of 300 to 600 microns.

In some aspects, the superabsorbent polymer material has a centrifuge retention capacity of 23g/g or more, as measured by the Centrifuge Retention Capacity Test. In other aspects, the superabsorbent polymer material has a free swell gel bed permeability of 100 Darcy or more, as measured by the Free Swell Gel Bed Permeability Test. In still other aspects, the superabsorbent polymer material has a superabsorbent material shake-out of less than 20%, as measured by the Oven Shake-Out Test.

In some aspects, the absorbent core further comprises a meltblown thermoplastic fiber and exhibits a Stretchability of at least 50% as measured by the Composite Stretchability Test, a Composite Permeability of at least 15 Darcy as measured by the Composite Permeability Test, at least one FIFE Intake Rate greater than 1 g/sec as measured by the Fluid Intake Rate Test, and a Composite Shakeout value of not more than 2% by weight as measured by the Composite Shakeout Test. The result is an absorbent composite which exhibits improved performance as well as greater comfort and confidence among the user.

The absorbent core of the present invention can be used on its own, or can be utilized as a component of an absorbent article. In addition, such articles can comprise a topsheet and/or a backsheet. In some aspects, at least the topsheet can be stretchable, while in other aspects, at least the backsheet can be stretchable. In still other aspects of the invention, the absorbent core can be stretchable. The absorbent article may also include other components, such as fluid wicking layers, intake layers, surge layers, distribution layers, transfer layers, barrier layers, wrapping layers and the like, as well as combinations thereof.

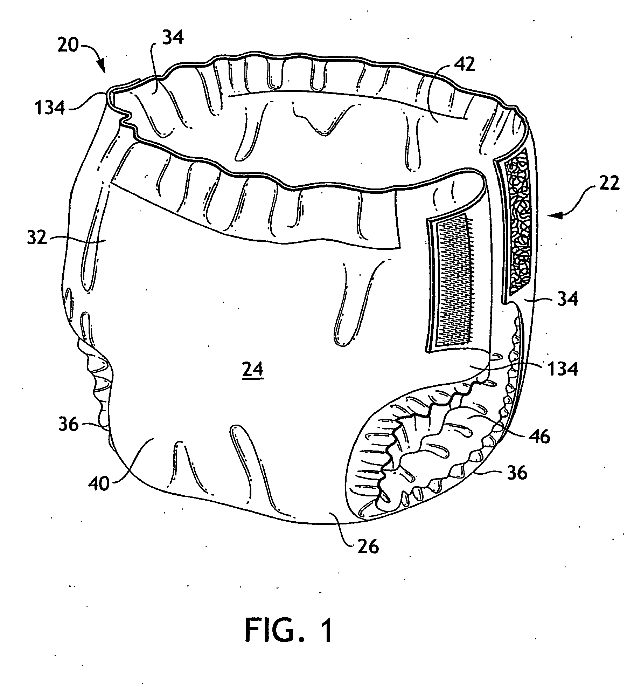

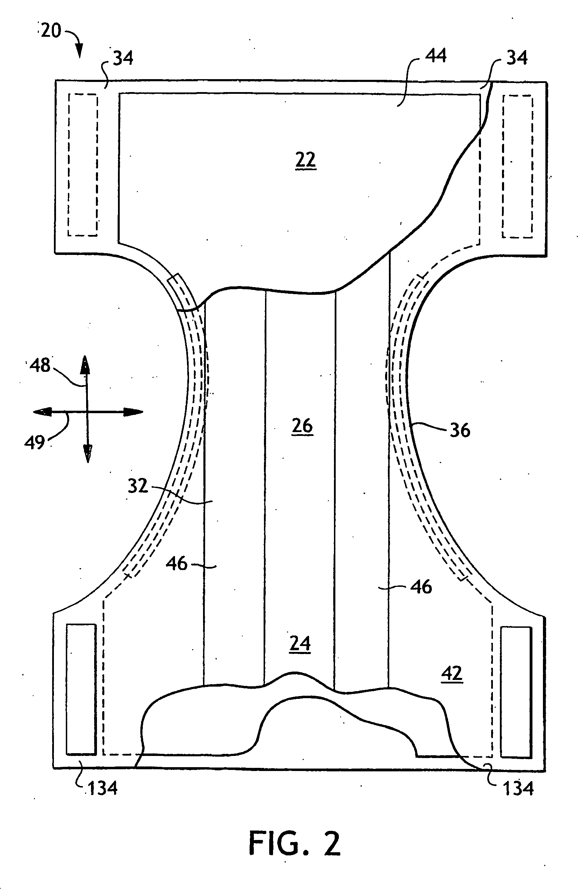

Referring to FIGs 1 and 2 for exemplary purposes, a training pant which may incorporate the present invention is shown. It is understood that the present invention is suitable for use with various other absorbent articles, including but not limited to other personal care absorbent articles, health/medical absorbent articles, household/industrial absorbent articles, and the like, without departing from the scope of the present invention.

Various materials and methods for constructing training pants are disclosed in PCT Patent Application WO 00/37009 published June 29, 2000 by A. Fletcher et al.; U.S. Patents 4,940,464 to Van Gompel et al.; 5.766,389 to Brandon et al., and 6.645,190 to Olson et. al..

FIG 1 illustrates a training pant in a partially fastened condition, and FIG 2 illustrates a training pant in an opened and unfolded state. The training pant defines a longitudinal direction 48 that extends from the front of the training pant when worn to the back of the training pant. Perpendicular to the longitudinal direction 48 is a lateral direction 49.

The pair of training pants defines a front region 22, a back region 24, and a crotch region 26 extending longitudinally between and interconnecting the front and back regions. The pant also defines an inner surface adapted in use (e.g., positioned relative to the other components of the pant) to be disposed toward the wearer, and an outer surface opposite the inner surface. The training pant has a pair of laterally opposite side edges and a pair of longitudinally opposite waist edges.

The illustrated pant 20 may include a chassis 32, a pair of laterally opposite front side panels 34 extending laterally outward at the front region 22 and a pair of laterally opposite back side panels 134 extending laterally outward at the back region 24.

The chassis 32 includes a backsheet 40 and a topsheet 42 that may be joined to the backsheet 40 in a superimposed relation therewith by adhesives, ultrasonic bonds, thermal bonds or other conventional techniques. The chassis 32 may further include an absorbent core 44 such as shown in FIG 2 disposed between the backsheet 40 and the topsheet 42 for absorbing fluid body exudates exuded by the wearer, and may further include a pair of containment flaps 46 secured to the topsheet 42 or the absorbent core 44 for inhibiting the lateral flow of body exudates.

The backsheet 40, the topsheet 42 and the absorbent core 44 may be made from many different materials known to those skilled in the art. All three layers, for instance, may be extensible and/or elastically extensible. Further, the stretch properties of each layer may vary in order to control the overall stretch properties of the product.

The backsheet 40, for instance, may be breathable and/or may be fluid impermeable. The backsheet 40 may be constructed of a single layer, multiple layers, laminates, spunbond fabrics, films, meltblown fabrics, elastic netting, microporous webs or bonded-carded-webs. The backsheet 40, for instance, can be a single layer of a fluid impermeable material, or alternatively can be a multilayered laminate structure in which at least one of the layers is fluid impermeable.

The backsheet 40 can be biaxially extensible and optionally biaxially elastic. Elastic non-woven laminate webs that can be used as the backsheet 40 include a non-woven material joined to one or more gatherable non-woven webs or films. Stretch Bonded Laminates (SBL) and Neck Bonded Laminates (NBL) are examples of elastomeric composites.

Examples of suitable nonwoven materials are spunbond-meltblown fabrics, spunbond-meltblown-spunbond fabrics, spunbond fabrics, or laminates of such fabrics with films, or other nonwoven webs. Elastomeric materials may include cast or blown films, meltblown fabrics or spunbond fabrics composed of polyethylene, polypropylene, or polyolefin elastomers, as well as combinations thereof. The elastomeric materials may include PEBAX elastomer (available from AtoFina Chemicals, Inc., a business having offices located in Philadelphia, Pennsylvania U.S.A.), HYTREL elastomeric polyester (available from Invista, a business having offices located in Wichita, Kansas U.S.A.), KRATON elastomer (available from Kraton Polymers, a business having offices located in Houston, Texas, U.S.A.), or strands of LYCRA elastomer (available from Invista), or the like, as well as combinations thereof. The backsheet 40 may include materials that have elastomeric properties through a mechanical process, printing process, heating process or chemical treatment. For example, such materials may be apertured, creped, neck-stretched, heat activated, embossed, and micro-strained, and may be in the form of films, webs, and laminates.

One example of a suitable material for a biaxially stretchable backsheet 40 is a breathable elastic film/nonwoven laminate, such as described in U.S. Patent 5,883,028, to Morman et al.. Examples of materials having two-way stretchability and retractability are disclosed in U.S. Patents 5,116,662 to Morman and 5,114,781 to Morman. These two patents describe composite elastic materials capable of stretching in at least two directions. The materials have at least one elastic sheet and at least one necked material, or reversibly necked material, joined to the elastic sheet at least at three locations arranged in a nonlinear configuration, so that the necked, or reversibly necked, web is gathered between at least two of those locations.

The topsheet 42 is suitably compliant, soft-feeling and non-irritating to the wearer's skin. The topsheet 42 is also sufficiently liquid permeable to permit liquid body exudates to readily penetrate through its thickness to the absorbent core 44. A suitable topsheet 42 may be manufactured from a wide selection of web materials, such as porous foams, reticulated foams, apertured plastic films, woven and non-woven webs, or a combination of any such materials. For example, the topsheet 42 may include a meltblown web, a spunbonded web, or a bonded-carded-web composed of natural fibers, synthetic fibers or combinations thereof. The topsheet 42 may be composed of a substantially hydrophobic material, and the hydrophobic material may optionally be treated with a surfactant or otherwise processed to impart a desired level of wettability and hydrophilicity.

The topsheet 42 may also be extensible and/or elastomerically extensible. Suitable elastomeric materials for construction of the topsheet 42 can include elastic strands, LYCRA elastics, cast or blown elastic films, nonwoven elastic webs, meltblown or spunbond elastomeric fibrous webs, as well as combinations thereof. Examples of suitable elastomeric materials include KRATON elastomers, HYTREL elastomers, ESTANE elastomeric polyurethanes (available from Noveon, a business having offices located in Cleveland, Ohio U.S.A.), or PEBAX elastomers. The topsheet 42 can also be made from extensible materials such as those described in U.S. Patent 6,552,245 to Roessler et al.. The topsheet 42 can also be made from biaxially stretchable materials as descried in U.S. Patent 6,641,134 filed to Vukos et al.

The article 20 can optionally further include a surge management layer which may be located adjacent the absorbent core 44 and attached to various components in the article 20 such as the absorbent core 44 or the topsheet 42 by methods known in the art, such as by using an adhesive. In general, a surge management layer helps to quickly acquire and diffuse surges or gushes of liquid that may be rapidly introduced into the absorbent structure of the article. The surge management layer can temporarily store the liquid prior to releasing it into the storage or retention portions of the absorbent core 44. Examples of suitable surge management layers are described in U.S. Patents 5,486,166 to Bishop et al.; 5,490,846 to Ellis et al.; and 5,820,973 to Dodge et al.. The article 20 can further comprise an absorbent core 44. The absorbent core 44 may have any of a number of shapes. For example, it may have a 2-dimensional or 3-dimensional configuration, and may be rectangular shaped, triangular shaped, oval shaped, race-track shaped, I-shaped, generally hourglass shaped, T-shaped and the like. It is often suitable for the absorbent core 44 to be narrower in the crotch portion 26 than in the rear 24 or front 22 portion(s). The absorbent core 44 can be attached in an absorbent article, such as to the backsheet 40 and/or the topsheet 42 for example, by bonding means known in the art, such as ultrasonic, pressure, adhesive, aperturing, heat, sewing thread or strand, autogenous or self-adhering, hook-and-loop, or any combination thereof.

In some aspects, the absorbent core 44 can have a significant amount of stretchability. For example, the absorbent core 44 can comprise a matrix of fibers which includes an operative amount of elastomeric polymer fibers. Other methods known in the art can include attaching superabsorbent materials to a stretchable film, utilizing a nonwoven substrate having cuts or slits in its structure, and the like.

The absorbent core 44 can be formed using methods known in the art. While not being limited to the specific method of manufacture, the absorbent core can utilize a meltblown process and can further be formed on a coform line. Exemplary meltblown processes are described in various patents and publications, including NRL Report 4364, "Manufacture of Super-Fine Organic Fibers" by V. A. Wendt, E. L. Boone and C. D. Fluharty; NRL Report 5265, "An Improved Device For the Formation of Super-Fine Thermoplastic Fibers" by K. D. Lawrence, R. T. Lukas and J. A. Young; and U.S. Patent Nos. 3,849,241 and 5,350,624.

To form "coform" materials, additional components are mixed with the meltblown fibers as the fibers are deposited onto a forming surface. For example, the superabsorbent materials of the present invention and fluff, such as wood pulp fibers, may be injected into the meltblown fiber stream so as to be entrapped and/or bonded to the meltblown fibers. Exemplary coform processes are described in U.S. Patent Nos. 4,100,324 to Anderson et al.; 4,587,154 to Hotchkiss et al.; 4,604,313 to McFarland et al.; 4,655,757 to McFarland et al.; 4,724,114 to McFarland et al.; 4,100,324 to Anderson et al.; and U.K Patent GB 2,151,272 to Minto et al.. Absorbent, elastomeric meltblown webs containing high amounts of superabsorbent are described in U.S. Patent No. 6,362,389 to D. J. McDowall, and absorbent, elastomeric meltblown webs containing high amounts of superabsorbent and low superabsorbent shakeout values are described in pending U.S. Patent Application 10/883174 to X. Zhang et al..

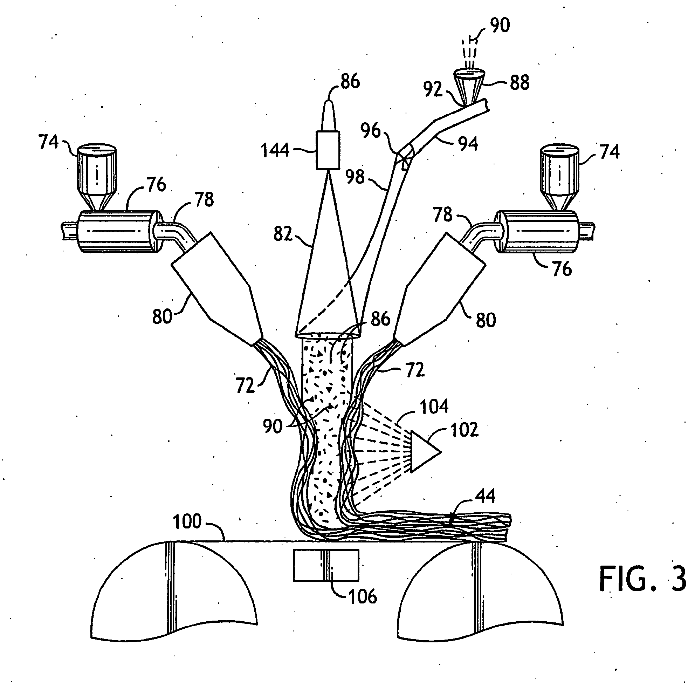

One example of a method of forming the absorbent core 44 of the present invention is illustrated in FIG 3. The dimensions of the apparatus in FIG 3 are described herein by way of example. Other types of apparatus having different dimensions and/or different structures may also be used to form the absorbent core 44. As shown in FIG 3, elastomeric material 72 in the form of pellets can be fed through two pellet hoppers 74 into two single screw extruders 76 that each feed a spin pump 78. The elastomeric material 72 may be a multicomponent elastomer blend available under the trade designation VISTMAXX 2370 from ExxonMobil Chemical Company (a business having offices located in Houston, Texas U.S.A.), as well as others mentioned herein. Each spin pump 78 feeds the elastomeric material 72 to a separate meltblown die 80. Each meltblown die 80 may have 12 holes per cm (30 holes per inch (hpi)). The die angle may be adjusted anywhere between 0 and 70 degrees from horizontal, and is suitably set at about 45 degrees. The forming height may be at a maximum of abot 40.6 cm (16 inches), but this restriction may differ with different equipment.

A chute 82 having a width of about 61 cm (0.4 inches) wide may be positioned between the meltblown dies 80. The depth, or thickness, of the chute 82 may be adjustable in a range from about 1.27 cm (0.5) to about 3.18 cm (1.25 inches), or from about 1.91 cm (0.75) to about 2.54 cm (1.0 inch). A picker 144 connects to the top of the chute 82. The picker 144 is used to fiberize the pulp fibers 86. The picker 144 may be limited to processing low strength or debonded (treated) pulps, in which case the picker 144 may limit the illustrated method to a very small range of pulp types. In contrast to conventional hammermills that use hammers to impact the pulp fibers repeatedly, the picker 144 uses small teeth to tear the pulp fibers 86 apart. Suitable pulp fibers 86 for use in the method illustrated in FIG 3 include those mentioned herein, such as NB480 (available from Weyerhaeuser Co., a business having offices located in Federal Way, Washington U.S.A.).

At an end of the chute 82 opposite the picker 144 is a superabsorbent material feeder 88. The feeder 88 pours the superabsorbent material 90 of the present invention into a hole 92 in a pipe 94 which then feeds into a blower fan 96. Past the blower fan 96 is a length of 10.2 cm (4-inch) diameter pipe 98 sufficient for developing a fully developed turbulent flow at about 1520 metres per minute (5,000 feet per minute), which allows the superabsorbent material 90 to become distributed. The pipe 98 widens from a 10.2 cm (4-inch) diameter to the 61 cm by 1.91 cm (24-inch by 0.75-inch) chute 82, at which point the superabsorbent material 90 mixes with the pulp fibers 86 and the mixture falls straight down and gets mixed on either side at an approximately 45-degree angle with the elastomeric material 72. The mixture of superabsorbent material 90, pulp fibers 86, and elastomeric material 72 falls onto a wire conveyor 100 moving from about 4.27 to about 10.7 metres per minute (about 14 to about 35 feet per minute). However, before hitting the wire conveyor 100, a spray boom 102 optionally sprays an aqueous surfactant mixture 104 in a mist through the mixture, thereby rendering the resulting absorbent core 44 wettable. The surfactant mixture 104 may be a 1:3 mixture of GLUCOPON 220 UP (available from Cognis Corporation having a place of business in Cincinnati, Ohio, U.S.A.) and AHCOVEL Base N-62 (available from Uniqema, having a place of business in New Castle, Delaware, U.S.A.). An under wire vacuum 106 is positioned beneath the conveyor 100 to assist in forming the absorbent core 44.

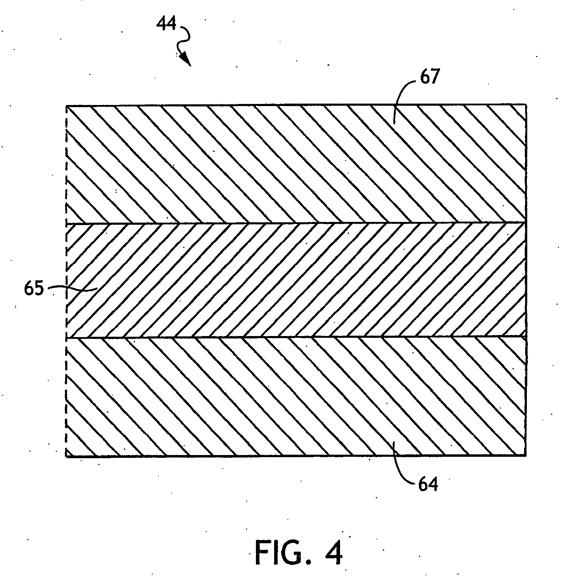

In general, the absorbent core 44 is often a unitary structure comprising a substantially uniform distribution of superabsorbent materials, fibers, and any other optional additives. However, referring to FIG 4, in some aspects, the absorbent core 44 of the present invention may be further enhanced through structural modifications when combined with superabsorbent materials of the present invention. For example, providing a layer 65 comprising substantially superabsorbent materials of the present invention sandwiched between layers 67 and 64 comprising substantially fluff fibers, such as NB480, can result in an absorbent core 44 having improved absorbent properties, such as fluid insult intake rate, when compared to a structure comprising a substantially uniform distribution of the superabsorbent materials and fluff fibers. Such layering can occur in the z-direction of the absorbent core 44 and may optionally cover the entire x-y area. However, the layers 65 through 64 need not be discreet from one another. For example, in some aspects, the z-directional middle portion 65 of the absorbent core need only contain a higher superabsorbent material percentage (e.g., at least about 10% by weight higher) than the top layer 67 and/or bottom layer 64 of the absorbent core 44. Desirably, the layers 65 through 64 are present in the area of the absorbent core 44 that is within an insult target zone.

The absorbent core 44 also includes absorbent material, such as superabsorbent material and/or fluff. Additionally, the superabsorbent material can be operatively contained within a matrix of fibers, such as polymeric fibers. Accordingly, the absorbent core 44 can comprise a quantity of superabsorbent material and/or fluff contained within a matrix of fibers. In some aspects, the amount of superabsorbent material in the absorbent core 44 can be at least 10% by weight of the core, such as at least 30%, or at least 60% by weight or at least 90%, or between 10% and 99% by weight of the core, or between 30% to 90% by weight of the core to provide improved benefits. Optionally, the amount of superabsorbent material can be at least about 95-percent by weight of the core. In other aspects, the absorbent core 44 can comprise about 35-percent or less by weight fluff, such as about 20-percent or less, or 10-percent or less by weight fluff.

It should be understood that the present invention is not restricted to use with superabsorbent materials and/or fluff. In some aspects, the absorbent core 44 may additionally or alternatively include materials such as surfactants, ion exchange resin particles, moisturizers, emollients, perfumes, natural fibers, synthetic fibers, fluid modifiers, odor control additives, and combinations thereof. Alternatively, the absorbent core 44 can include a foam.

In order to function well, the absorbent core 44 can have certain desired properties to provide improved performance as well as greater comfort and confidence among the user. For instance, the absorbent core 44 can have corresponding configurations of absorbent capacities, densities, basis weights and/or sizes which are selectively constructed and arranged to provide desired combinations of absorbency properties such as liquid intake rate, absorbent capacity, liquid distribution or fit properties such as shape maintenance and aesthetics. Likewise, the components can have desired wet to dry strength ratios, mean flow pore sizes, permeabilities and elongation values.

As mentioned above, the absorbent core 44 can optionally include elastomeric polymer fibers. The elastomeric material of the polymer fibers may include an olefin elastomer or a non-olefin elastomer, as desired. For example, the elastomeric fibers can include olefinic copolymers, polyethylene elastomers, polypropylene elastomers, polyester elastomers, polyisoprene, cross-linked polybutadiene, diblock, triblock, tetrablock, or other multi-block thermoplastic elastomeric and/or flexible copolymers such as block copolymers including hydrogenated butadiene-isoprene-butadiene block copolymers; stereoblock polypropylenes; graft copolymers, including ethylene-propylene-diene terpolymer or ethylene-propylene-diene monomer (EPDM) rubber, ethylene-propylene random copolymers (EPM), ethylene propylene rubbers (EPR), ethylene vinyl acetate (EVA), and ethylene-methyl acrylate (EMA); and styrenic block copolymers including diblock and triblock copolymers such as styrene-isoprene-styrene (SIS), styrene-butadiene-styrene (SBS), styrene-isoprene-butadiene-styrene (SIBS), styrene-ethylene/butylene-styrene (SEBS), or styrene-ethylene/propylene-styrene (SEPS), which may be obtained from Kraton Inc. under the trade designation KRATON elastomeric resin or from Dexco, a division of ExxonMobil Chemical Company under the trade designation VECTOR (SIS and SBS polymers); blends of thermoplastic elastomers with dynamic vulcanized elastomer-thermoplastic blends; thermoplastic polyether ester elastomers; ionomeric thermoplastic elastomers; thermoplastic elastic polyurethanes, including those available from Invista Corporation under the trade name LYCRA polyurethane, and ESTANE available from Noveon, Inc., a business having offices located in Cleveland, Ohio U.S.A.; thermoplastic elastic polyamides, including polyether block amides available from AtoFina Chemicals, Inc. (a business having offices located in Philadelphia, Pennsylvania U.S.A.) under the trade name PEBAX; polyether block amide; thermoplastic elastic polyesters, including those available from E. I. Du Pont de Nemours Co.; under the trade name HYTREL, and ARNITEL from DSM Engineering Plastics (a business having offices located in Evansville, Indiana, U.S.A.) and single-site or metallocene-catalyzed polyolefins having a density of less than about 0.89 grams/cubic centimeter, available from Dow Chemical Co. (a business having offices located in Freeport, Texas U.S.A.) under the trade name AFFINITY; and combinations thereof.

As used herein, a tri-block copolymer has an ABA structure where the A represents several repeat units of type A, and B represents several repeat units of type B. As mentioned above, several examples of styrenic block copolymers are SBS, SIS, SIBS, SEBS and SEPS. In these copolymers the A blocks are polystyrene and the B blocks are a rubbery component. Generally, these triblock copolymers have molecular weights that can vary from the low thousands to hundreds of thousands, and the styrene content can range from 5% to 75% based on the weight of the triblock copolymer. A diblock copolymer is similar to the triblock, but is of an AB structure. Suitable diblocks include styrene-isoprene diblocks, which have a molecular weight of approximately one-half of the triblock molecular weight having the same ratio of A blocks to B blocks.

In desired arrangements, the polymer fibers can include at least one material selected from the group consisting of styrenic block copolymers, elastic polyolefin polymers and co-polymers and EVA/EMA type polymers.

In some particular arrangements, for example, the elastomeric material of the polymer fibers can include various commercial grades of low crystallinity, lower molecular weight metallocene polyolefins, available from ExxonMobil Chemical Company (a company having offices located in Houston, Texas, U.S.A.) under the VISTAMAXX trade designation. Some VISTAMAXX materials are believed to be metallocene propylene ethylene co-polymer. For example, in one aspect the elastomeric polymer can be VISTAMAXX PLTD 2210. In other aspects, the elastomeric polymer can be VISTAMAXX PLTD 1778. In a particular aspect, the elastomeric polymer is VISTAMAXX 2370. Another optional elastomeric polymer is KRATON blend G 2755 from Kraton Inc. The KRATON material is believed to be a blend of styrene ethylene-butylene styrene polymer, ethylene waxes and tackifying resigns.

In some aspects, the elastomeric polymer fibers can be produced from a polymer material having a selected melt flow rate (MFR). In a particular aspect, the MFR can be up to a maximum of about 300. Alternatively, the MFR can be up to about 230 or 250. In another aspect, the MFR can be a minimum of not less than about 9, or not less than 20. The MFR can alternatively be not less than about 50 to provide desired performance. The described melt flow rate has the units of grams flow per 10 minutes (g/10 min). The parameter of melt flow rate is well known, and can be determined by conventional techniques, such as by employing test ASTM D 1238 70 "extrusion plastometer" Standard Condition "L" at 230°C and 2.16 kg applied force.

As referenced above, the polymer fibers of the absorbent core 44 can include an amount of a surfactant. The surfactant can be combined with the polymer fibers of the absorbent core in any operative manner. Various techniques for combining the surfactant are conventional and well known to persons skilled in the art. For example, the surfactant may be compounded with the polymer employed to form a meltblown fiber structure. In a particular feature, the surfactant may be configured to operatively migrate or segregate to the outer surface of the fibers upon the cooling of the fibers. Alternatively, the surfactant may be applied to or otherwise combined with the polymer fibers after the fibers have been formed.

The polymer fibers can include an operative amount of surfactant, based on the total weight of the fibers and surfactant. In some aspects, the polymer fibers can include at least a minimum of about 0.1 % by weight surfactant, as determined by water extraction. The amount of surfactant can alternatively be at least about 0.15% by weight, and can optionally be at least about 0.2% by weight to provide desired benefits. In other aspects, the amount of surfactant can be generally not more than a maximum of about 2% by weight, such as not more than about 1% by weight, or not more than about 0.5% by weight to provide improved performance.

If the amount of surfactant is outside the desired ranges, various disadvantages can occur. For example, an excessively low amount of surfactant may not allow fibers, such as hydrophobic meltblown fibers, to wet with the absorbed fluid. In contrast, an excessively high amount of surfactant may allow the surfactant to wash off from the fibers and undesirably interfere with the ability of the absorbent core to transport fluid, or may adversely affect the attachment strength of the absorbent core to the absorbent article. Where the surfactant is compounded or otherwise internally added to the polymer fibers, an excessively high level of surfactant can create conditions that cause poor formation of the polymer fibers and interfiber bonds.

In some configurations, the surfactant can include at least one material selected from the group that includes polyethylene glycol ester condensates and alkyl glycoside surfactants. For example, the surfactant can be a GLUCOPON surfactant, available from Cognis Corporation, which can be composed of 40-percent water, and 60-percent d-glucose, decyl, octyl ethers and oligomerics.

In other aspects of the invention, the surfactant can be in the form of a sprayed-on surfactant comprising a water/surfactant solution which includes 16 liters of hot water (about 45 °C to 50 °C) mixed with 0.20 kg of GLUCOPON 220 UP surfactant available from Cognis Corporation and 0.36 kg of AHCHOVEL Base N-62 surfactant available from Uniqema. When employing a sprayed-on surfactant, a relatively lower amount of sprayed-on surfactant may be desirable to provide the desired containment of the superabsorbent material. Excessive amounts of the fluid surfactant may hinder the desired attachment of the superabsorbent material to the molten, elastomeric meltblown fibers, for example.

An example of an internal surfactant or wetting agent that can be compounded with the elastomeric fiber polymer can include a MAPEG DO 400 PEG (polyethylene glycol) ester, available from BASF (a business having offices located in Freeport, Texas, U.S.A.). Other internal surfactants can include a polyether, a fatty acid ester, a soap or the like, as well as combinations thereof.

As referenced above, the absorbent core 44 can optionally include fluff, such as cellulosic fibers. Such cellulosic fibers may include, but are not limited to, chemical wood pulps such as sulfite and sulfate (sometimes called Kraft) pulps, as well as mechanical pulps such as ground wood, thermomechanical pulp and chemithermomechanical pulp. More particularly, the pulp fibers may include cotton, other typical wood pulps, cellulose acetate, debonded chemical wood pulp, and combinations thereof. Pulps derived from both deciduous and coniferous trees can be used. Additionally, the cellulosic fibers may include such hydrophilic materials as natural plant fibers, milkweed floss, cotton fibers, microcrystalline cellulose, microfibrillated cellulose, or any of these materials in combination with wood pulp fibers. Suitable cellulosic fluff fibers can include, for example, NB480 (available from Weyerhaeuser Co.); NB416, a bleached southern softwood Kraft pulp (available from Weyerhaeuser Co.); CR 54, a bleached southern softwood Kraft pulp (available from Bowater Inc., a business having offices located in Greenville, South Carolina U.S.A).; SULPHATATE HJ, a chemically modified hardwood pulp (available from Rayonier Inc., a business having offices located in Jesup, Georgia U.S.A.); NF 405, a chemically treated bleached southern softwood Kraft pulp (available from Weyerhaeuser Co.); and CR 1654, a mixed bleached southern softwood and hardwood Kraft pulp (available from Bowater Inc.)

As reference above, the absorbent core 44 also includes a desired amount of superabsorbent material of the present invention. Superabsorbent materials typically are polymers of unsaturated carboxylic acids or derivatives thereof. These polymers are rendered water insoluble, but water swellable, by crosslinking the polymer with a di- or poly-functional internal crosslinking agent. These internally crosslinked polymers are at least partially neutralized and contain pendant anionic carboxyl groups on the polymer backbone that enable the polymer to absorb aqueous fluids, such as body fluids.

In general, superabsorbent materials are manufactured by known polymerization techniques, preferably by polymerization in aqueous solution by gel polymerization. The products of this polymerization process are aqueous polymer gels (i.e., superabsorbent hydrogels) that are reduced in size to small particles by mechanical forces, then dried using drying procedures and apparatus known in the art. The drying process is followed by pulverization of the resulting superabsorbent material to the desired particle size.

To improve the fluid absorption profile, superabsorbent materials may be optimized with respect to one or more of absorption capacity, absorption rate, acquisition time, gel strength, and/or permeability. Optimization allows for a reduction in the amount of fluff fiber used in an absorbent article, which results in a thinner article. However, it is very difficult to maximize all of these absorption profile properties simultaneously.

One method of optimizing the fluid absorption profile of superabsorbent materials is to provide materials of a predetermined particle size distribution. In particular, particles that are too small in size swell after absorbing a fluid and can block the absorption of further fluid. Particles that are too large in size have a reduced surface area which decreases the rate of absorption.

Therefore, the particle size distribution of the superabsorbent materials is such that fluid permeability, absorption, and retention by the superabsorbent materials are maximized. Any subsequent process that agglomerates the superabsorbent materials to provide oversized particles should be avoided. In particular, agglomeration of superabsorbent materials increases apparent particle size, which reduces the surface area of the superabsorbent materials, and in turn adversely affects absorption of an aqueous fluid by the superabsorbent materials.

The superabsorbent materials of the present invention are directed to overcoming problems encountered in improving the absorption profile of the superabsorbent materials because improving one property often is detrimental to a second property. The present superabsorbent materials maintain the conflicting properties of a high Centrifuge Retention Capacity (CRC) and an excellent permeability, and can provide for an absorbent article with improved fluid intake rate.

In order to use an increased amount of superabsorbent materials and a decreased amount of fluff in the absorbent core 44, it is important to maintain a high liquid permeability. In particular, the permeability of a superabsorbent hydrogel layer formed by swelling in the presence of a fluid is very important to overcome the problem of leakage from the product. A lack of permeability directly impacts the ability of superabsorbent hydrogel layers to acquire and distribute such fluids.

The superabsorbent material comprises a superabsorbent polymer material which includes a crosslinked superabsorbent polymer comprising: a) from 55 to 99.9% by weight of polymerizable unsaturated acid group containing monomer, based on the weight of the dry superabsorbent polymer material; b) from 0.001 to 5% by weight of internal crosslinking agent based on the weight of the polymerizable unsaturated acid group containing monomer, wherein elements a) and b) are polymerized and prepared into superabsorbent polymer particles. The surfaces of these superabsorbent polymer particles can be surface treated with: i) from 0.00.1 to 5% by weight of surface crosslinking agent; ii) from 0.01 to 10% by weight of a penetration modifier, iii) from 0 to 5% by weight of a multivalent metal salt; iv) from 0.01 to 5% by weight of an insoluble, inorganic powder, v) from 0 to 2% by weight of a surface active agent, and vi) from 0.01 to 5% by weight of a thermoplastic polymer, comprising maleated polypropylene each based on the weight of the dry superabsorbent polymer material. The surface treated superabsorbent polymer particles are then heat-treated. The superabsorbent particles further comprise a coating of from 0.01 to 5% by weight of a cationic polymer, based on the superabsorbent material.

The superabsorbent polymer has a degree of neutralization of more than 25%. The superabsorbent polymer material can also have a centrifuge retention capacity (CRC) of from 25 g/g or more, as measured by the Centrifuge Retention Capacity Test; a free swell gel bed permeability (GBP) of at least 100 Darcy, such as at least 130 Darcy or at least 160 Darcy, or at least 200 Darcy, as measured by the Free Swell Gel Bed Permeability Test; and a superabsorbent material shake-out of less than 20%, such as less than 16%, as measured by the Oven Shake-Out Test.

As referenced above, the superabsorbent polymer material of the present invention Is obtained by the initial polymerization of from 55 to 99.9% by weight of a polymerizable unsaturated acid group containing monomer. A suitable monomer includes any of those containing carboxyl groups, such as acrylic acid, methacrylic acid or 2-acrylamido-2-methylpropanesulfonic acid, or mixtures thereof. It is desirable for at least about 50% by weight, such as at least about 75% by weight, of the acid groups to be carboxyl groups. The acid groups are neutralized to the extent of at least 25-mol%, that is, the acid groups are desirably present as sodium, potassium or ammonium salts. In some aspects, the degree of neutralization can be at least about 50-mol%. In some aspects, it is desirable to utilize polymers obtained by polymerization of acrylic acid or methacrylic acid, the carboxyl groups of which are neutralized to the extent of 50-80-mol%, in the presence of internal crosslinking agents.

In some aspects, the suitable monomer that can be copolymerized with the ethylenically unsaturated monomer may include, but is not limited to, acrylamide, methacrylamide, hydroxyethyl acrylate, dimethylaminoalkyl (meth)-acrylate, ethoxylated (meth)-acrylates, dimethylaminopropylacrylamide or acrylamidopropyltrimethylammonium chloride. Such monomer may be present In a range of 0 to 40% by weight of the copolymerized monomer.

As referenced above, the superabsorbent polymer material of the invention also includes internal crosslinking agents. The internal crosslinking agent has at least two ethylenically unsaturated double bonds, or one ethylenically unsaturated double bond and one functional group which is reactive toward acid groups of the polymerizable unsaturated acid group containing monomer, or several functional groups which are reactive toward acid groups, and can be used as the internal crosslinking component and is desirably present during the polymerization of the polymerizable unsaturated acid group containing monomer.

Examples of internal crosslinking agents include, but are not limited to, aliphatic unsaturated amides, such as methylenebisacryl- or -methacrylamide or ethylenebisacrylamide; aliphatic esters of polyols or alkoxylated polyols with ethylenically unsaturated acids, such as di(meth)acrylates or tri(meth)acrylates of butanediol or ethylene glycol, polyglycols or trimethylolpropane; di- and triacrylate esters of trimethylolpropane which may be oxyalkylated, desirably ethoxylated, with 1 to 30 moles of alkylene oxide; acrylate and methacrylate esters of glycerol and pentaerythritol and of glycerol and pentaerythritol oxyethylated with desirably 1 to 30 mol of ethylene oxide; allyl compounds, such as allyl (meth)acrylate, alkoxylated allyl (meth)acrylate reacted with desirably 1 to 30 mol of ethylene oxide, triallyl cyanurate, triallyl isocyanurate, maleic acid diallyl ester, poly-allyl esters, tetraallyloxyethane, triallylamine, tetraallylethylenediamine, diols, polyols, hydroxy allyl or acrylate compounds and allyl esters of phosphoric acid or phosphorous acid; and monomers which are capable of crosslinking, such as N-methylol compounds of unsaturated amides, such as of methacrylamide or acrylamide, and the ethers derived therefrom. Ionic crosslinkers such as multivalent metal salts may also be employed. Mixtures of the crosslinking agents mentioned can also be employed. The content of the internal crosslinking agents is from 0.01 to 5% by weight, such as 0.1 to 3% by weight based on the total amount of the polymerizable unsaturated acid group containing monomer.

In some aspects, initiators can be used for initiation of the free-radical polymerization. Suitable initiators include, but are not limited to, azo or peroxo compounds, redox systems or UV initiators, sensitizers, and/or radiation.

After polymerization, the superabsorbent polymer is generally formed into particles. The superabsorbent polymer particles are surface crosslinked after polymerization. In general, surface crosslinking is a process that increases the crosslink density of the polymer matrix in the vicinity of the superabsorbent particle surface with respect to the crosslinking density of the particle interior. The superabsorbent polymer particles are typically surface crosslinked by the addition of a surface crosslinking agent. In some particular aspects, desirable surface crosslinking agents include chemicals with one or more functional groups, which are reactive toward pendant groups of the polymer chains, typically the acid groups. The surface crosslinking agent may be present in an amount of from 0.01 to 5% by weight, such as from 0.1 to 3% by weight, based on the weight of the dry superabsorbent polymer material. A heating step is preferred after addition of the surface crosslinking agent.

In one particular aspect, the particulate superabsorbent polymer is coated or surface treated with an alkylene carbonate, followed by heating, to effect surface crosslinking, which can improve the surface crosslinking density and the gel strength characteristics. More specifically, the surface crosslinking agent is coated onto the particulate by mixing the polymer with an aqueous alcoholic solution of the alkylene carbonate surface crosslinking agent. The amount of alcohol is determined by the solubility of the alkylene carbonate and is kept as low as possible for technical reasons, for instance protection against explosions. Suitable alcohols are methanol, ethanol, isopropanol, butanol, or butyl glycol, as well as mixtures of these alcohols. In some aspects, the solvent desirably Is water, which typically is used in an amount of 0.3 to 5.0% by weight, based on the weight of the dry superabsorbent polymer material. In other aspects, the alkylene carbonate surface crosslinking agent is dissolved in water without any alcohol. In still other aspects, the alkylene carbonate surface crosslinking agent may be applied from a powder mixture, for example, with an inorganic carrier material, such as $SiO_2$, or in a vapor state by sublimation of the alkylene carbonate.

To achieve the desired surface crosslinking properties, the alkylene carbonate should be distributed evenly on the superabsorbent polymer particle, to form surface treated superabsorbent polymer particles. For this purpose, mixing is effected In suitable mixers known in the art, such as fluidized bed mixers, paddle mixers, rotary drum mixers, or twin-worm mixers. It is also possible to carry out the coating of the particulate superabsorbent polymer material during one of the process steps in the production of the superabsorbent polymer. In one particular aspect, a suitable process for this purpose is the inverse suspension polymerization process.

The thermal treatment, which follows the coating treatment, is carried out as follows. In general, the thermal treatment is at a temperature of from about 100 to about 300°C. Lower temperatures are possible if highly reactive epoxide crosslinking agents are used. However, if alkylene carbonates are used, then the thermal treatment is suitably at a temperature of from about 150 to about 250˚C. In this particular aspect, the treatment temperature depends on the dwell time and the kind of alkylene carbonate. For example, at a temperature of about 150˚C, the thermal treatment is carried out for one hour or longer. In contrast, at a temperature of about 250˚C, a few minutes (e.g., about 0.5 to about 5 minutes) are sufficient to achieve the desired surface cross-linking properties. The thermal treatment may be carried out in conventional dryers or ovens known in the art.

While particles may be used by way of example of a physical form of superabsorbent polymer material, the invention is not limited to this form and is applicable to other forms such as fibers, foams, films, beads, rods and the like, as discussed above. In some aspects, when the superabsorbent polymer material exists in granule form, it is desirable that these particles have a size of from about 150 $\mu$m to about 850 $\mu$m based on the sieving process that is well know in the superabsorbent industry.

In some aspects, The superabsorbent polymer material according to the invention includes from 0.01 to 10% by weight, such as from 0.01 to 5% by weight, of a penetration modifier (based on the dry superabsorbent polymer material) that is added immediately before, during or immediately after the surface crosslinking agent. Examples of penetration modifiers include compounds which alter the penetration depth of surface-modifying agents into the superabsorbent polymer material by changing the viscosity, surface tension, ionic character or adhesion of the agents or medium in which these agents are applied. Suitable penetration modifiers include, but are not limited to, polyethylene glycols, tetraethylene glycol dimethyl ether, monovalent metal salts, surfactants, and water soluble polymers or blends thereof. It is noted that in some aspects, the nature and relation amount of the median or solvent itself used to apply the surface crosslinker agent can also act as a penetration modifier.

In some aspects, the superabsorbent polymer material according to the invention can include from 0 to 5% by weight, such as 0.1 to 5% by weight, of a multivalent metal salt (based on the weight of the mixture) on the surface of the polymer particles. In some particular aspects, the multivalent metal salt is desirably water soluble. Examples of suitable metal cations include the cations of Al, Ca, Fe, Zr, Mg and Zn. In one particular aspect, the metal cation has a valence of at least +3, with Al being most desirable. Example of suitable anions in the multivalent metal salt include halides, chlorohydrates, sulfates, nitrates, phosphates and acetates. In particular aspects, chlorohydrates and sulfates are more desirable. In one particular aspect, sulfates are the most desirable. For example, aluminum sulfate may be a desirable multivalent metal salt and is readily commercially available. A suitable form of aluminum sulfate is hydrated aluminum sulfate, such as aluminum sulfate having from 12 to 14 waters of hydration, for example. In addition to the salts discussed above, mixtures of multivalent metal salts can also be employed.

The polymer and multivalent metal salt can be suitably mixed by dry blending, or by mixing in solution, using means well known to those skilled in the art. In some aspects, aqueous solutions or dispersions are desirable. With dry blending, a binder may be employed in an amount sufficient to ensure that a substantially uniform mixture of the salt and the superabsorbent polymer is maintained. The binder may be water or a nonvolatile organic compound having a boiling point of at least about 150˚C. Examples of binders include water, polyols such as propylene glycol, glycerin and poly(ethylene glycol).

The superabsorbent polymer material according to the invention includes from 0.01 to 5% by weight of water-insoluble inorganic powder, such as from 0.1 to 4% by weight of water-insoluble inorganic powder, based on the weight of the dry superabsorbent polymer material. Examples of water-insoluble, inorganic powders include silica, fumed silica, silicon dioxide, silicic acid, silicates, titanium dioxide, aluminum oxide, magnesium oxide, zinc oxide, talc, calcium phosphate, clays, diatomataceous earth, zeolites, bentonite, kaolin, hydrotalcite, activated clays, etc. The water-insoluble inorganic powder additive may be a single compound or a mixture of compounds selected from the above list. In some particular aspects, microscopic noncrystalline silicon dioxide or aluminum oxide is desirable. In some aspects, the particle diameter of the inorganic powder can be 1,000 $\mu$m or smaller, such as 100 $\mu$m or smaller.

In some aspects, the superabsorbent polymer material according to the invention can also include the addition of from 0 to about 5% by weight of a surfactant to the polymer particle surface, based on the weight of the dry superabsorbent polymer material. In some particular aspects, the surfactants can be added immediately prior to, during or immediately after the surface crosslinking step.

Examples of suitable surfactants include anionic, non-ionic, cationic and amphoteric surface active agents, such as fatty acid salts, coco amines and amides and their salts, alkylsulfuric ester salts, alkylbenzene sulfonic acid salts, dialkyl sulfo-succinate, alkyl phosphate salt, and polyoxyethylene alkyl sulfate salt; polyoxyethylene alkyl ether, polyoxyethylene alkyl phenol ether, polyoxyethylene fatty acid ester, sorbitan fatty acid ester, polyoxy sorbitan fatty acid ester, polyoxyethylene alkylamine, fatty acid esters, and oxyethylene-oxypropylene block polymer; alkyl amine salts, quaternary ammonium salts; and lauryl dimethylamine oxide. However, suitable surfactants are not restricted to those mentioned above. Such surfactants may be used individually, or in combination.

In some aspects, the superabsorbent polymer material may also include from 0 to about 30% by weight, such as about 0.1 to about 5% by weight of water-soluble polymers based on the dry superabsorbent polymer material, such as partly or completely hydrolyzed polyvinyl acetate, polyvinylpyrrolidone, starch or starch derivatives, polyglycols, polyethylene oxides, polypropylene oxides, or polyacrylic acids. In some particular aspects, the water-soluble polymers are desirably in polymerized-in form.

The superabsorbent polymer material of the present invention includes from 0.01 to 5% by weight of a thermoplastic polymer (based on the dry superabsorbent polymer material) having a thermoplastic melt temperature wherein the thermoplastic polymer is applied onto the particle surface coincident with or followed by a temperature of the treated superabsorbent polymer particle at about the thermoplastic melt temperature. In some particular aspects, the thermoplastic polymer desirably is a polymer that may be in a solid, emulsion, suspension, colloidal, or solubilized state or combinations thereof. The thermoplastic polymer comprises maleated polypropylene (MPP) and may further comprise polyolefin, polyethylene, polyester, polyamide, polyurethane, styrene polybutadiene, linear low density polyethylene (LLDPE), ethylene acrylic acid copolymer (EAA), ethylene alkyl methacrylate copolymer (EMA), polypropylene (PP), maleated polypropylene, ethylene vinyl acetate copolymer (EVA), polyester, polyamide, and blends of all families of polyolefins, such as blends of PP, EVA, EMA, EEA, EBA, HDPE, MDPE, LDPE, LLDPE, and/or VLDPE, may also be advantageously employed. The term polyolefin as used herein is defined above. In particular aspects, ethylene acrylic acid copolymer polyester and EVA are preferred thermoplastic polymers for use in the present invention. The thermoplastic polymer may be functionalized to have additional benefits such as water solubility or dispersability.

In some aspects, the superabsorbent polymer material can also include from 0 to about 2% by weight of dedusting agents, such as hydrophilic and hydrophobic dedusting agents, based on the dry superabsorbent polymer material. Suitable dedusting agents include, but are not limited to, those described in U.S. Patents 6,090,875 and 5,994,440.

In some aspects, additional surface additives may optionally be employed with the superabsorbent polymer particles, such as odor-binding substances, such as cyclodextrins, zeolites, inorganic or organic salts and similar materials; anti-caking additives; flow modification agents and the like. In addition, surface additives may be employed that perform several roles during surface modifications. For example, a single additive may be a surfactant, viscosity modifier and can react to crosslink polymer chains.

The present invention further includes post treating the superabsorbent polymer material after surface treatment with from 0.1 to 5% by weight, of a cationic polymer, based on the dry superabsorbent polymer material. A cationic polymer as used herein refers to a polymer or mixture of polymers comprising a functional group or groups having a potential of becoming positively charged ions upon ionization in an aqueous solution. Suitable functional groups for a cationic polymer include, but are not limited to, primary, secondary, or tertiary amino groups, imino groups, imido groups, amido groups, and quaternary ammonium groups. Examples of synthetic cationic polymers include the salts or partial salts of poly(vinyl amines), poly(allylamines), poly(ethylene imine), poly(amino propanol vinyl ethers), poly(acrylamidopropyl trimethyl ammonium chloride), poly(diallyldimethyl ammonium chloride). Examples of natural based cationic polymers include partially deacetylated chitin, chitosan and chitosan salts. Synthetic polypeptides such as polyasparagins, polylysines, polyglutamines, polyarginines are also suitable cationic polymers.

In some aspects, the superabsorbent polymer material of the present invention may be, after a heat treatment step, treated with water so that the superabsorbent polymer material has a water content of up to about 10% by weight of the superabsorbent polymer material. This water may be added with one or more of the surface additives from above added to the superabsorbent polymer. The amount of water content, measured as "% moisture," can be measured as follows: Weigh 4.5-5.5 grams of superabsorbent polymer material (SAP) accurately in a pre-weighed aluminum weighing pan; '2) place the SAP and pan into a standard lab oven preheated to 150˚C for 30 minutes; 3) remove and re-weigh the pan and contents; and 4) calculate the percent moisture using the following formula:

$$\% \text{ Moisture} = \{ ((\text{pan wt.} + \text{initial SAP wt.}) - (\text{pan wt.} + \text{dried SAP})) * 100 \} / \text{dried SAP wt.}$$

The superabsorbent polymer material according to the invention are desirably prepared by two methods. The material can be prepared continuously or discontinuously in a large-scale industrial manner, with the after-crosslinking according to the invention being carried out accordingly.

According to one method, the partially neutralized monomer, such as acrylic acid, is converted into a gel by free-radical polymerization in aqueous solution in the presence of crosslinking agents and any further components, and the gel is comminuted, dried, ground and sieved off to the desired particle size. This solution polymerization can be carried out continuously or discontinuously.

According to another method, inverse suspension and emulsion polymerization can be used for preparation of the products according to the invention. According to these processes, an aqueous, partly neutralized solution of monomer, such as acrylic acid, is dispersed in a hydrophobic, organic solvent with the aid of protective colloids and/or emulsifiers and the polymerization is started by free radical initiators. The internal crosslinking agents may be either dissolved in the monomer solution and metered in together with this, or are added separately and optionally during the polymerization. The addition of a water-soluble polymer as the graft base optionally takes place via the monomer solution or by direct introduction into the oily phase. The water is then removed azeotropically from the mixture and the polymer is filtered off and optionally dried. Internal crosslinking can be carried out by polymerizing-in a polyfunctional crosslinking agent dissolved in the monomer solution and/or by reaction of suitable crosslinking agents with functional groups of the polymer during the polymerization steps.

The result of these methods is a superabsorbent pre-product. A superabsorbent pre-product as used herein is produced by repeating all of the steps for making the superabsorbent up to and including drying the material and coarse grinding in a crusher and removing particles greater than about 850 microns and smaller than about 150 microns.

The superabsorbent polymer material according to the invention can be employed in many products including sanitary towels, diapers or in wound coverings, for example. The superabsorbent polymer materials have the ability to rapidly absorb large amounts of menstrual blood, urine or other body fluids. Since the agents according to the invention retain the absorbed liquids even under pressure and additionally are capable of distributing further liquid within the construction in the swollen state, they are more desirably employed in higher concentrations, with respect to any hydrophilic fiber material, such as fluff, when compared to conventional current superabsorbent materials. They are also suitable for use as a homogeneous superabsorbent layer without fluff content within the absorbent article construction, as a result of which particularly thin articles are possible. The polymers are furthermore suitable for use in hygiene articles (incontinence products) for adults.

The preparation of laminates in the broadest sense, and of extruded and coextruded, wet- and dry-bonded, as well as subsequently bonded, structures are possible as further preparation processes. A combination of these possible processes with one another is also possible.

The superabsorbent polymer material according to the invention may also be employed in absorbent articles that are suitable for further uses. In particular, the superabsorbent polymer material of this invention can be used in absorbent material for absorbents for water or aqueous liquids, preferably in constructions for absorption of body fluids, in foamed and non-foamed sheet-like structures, in packaging materials, in constructions for plant growing, as soil improvement agents or as active compound carriers. For this, they are processed to a web by mixing with paper or fluff or synthetic fibers or by distributing the superabsorbent polymers between substrates of paper, fluff or non-woven textiles or by processing into carrier materials.

They are further suited for use in absorbent material such as wound dressings, packaging, agricultural absorbents, food trays and pads, and the like.

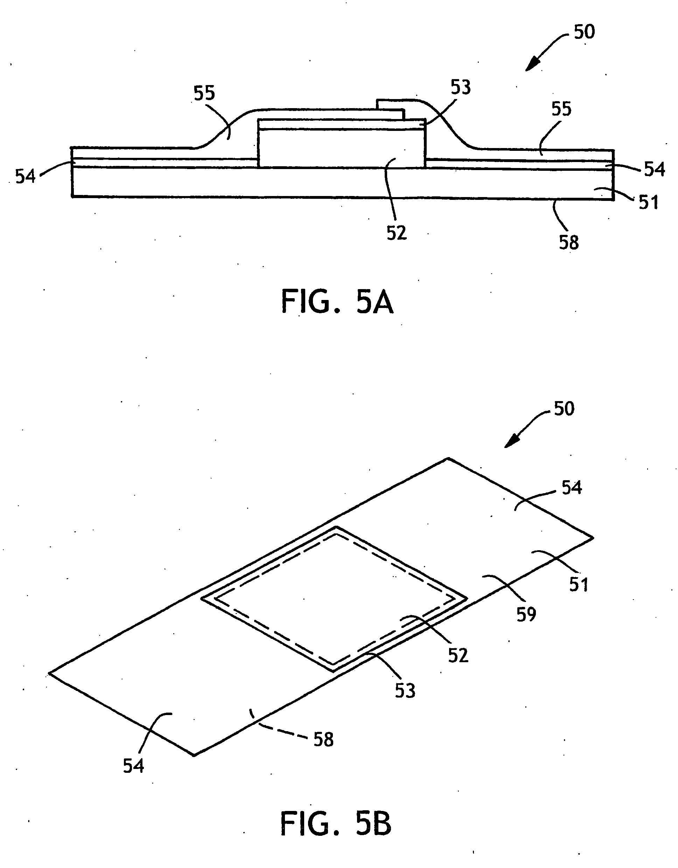

In addition to the absorbent articles described above, the absorbent articles of the present invention may be used as an absorbent bandage. Attention is directed to FIGS 5A and 5B, which show a possible configuration for a bandage of the present invention. FIG 5A shows a cross-section view of the absorbent bandage with optional layers described below. FIG 5B shows a perspective view of the bandage of the present invention with some of the optional or removable layers not being shown. The absorbent bandage 50 has a strip 51 of material having a body-facing side 59 and a second side 58 which is opposite the body-facing side. The strip is essentially a backsheet and is desirably prepared from the same materials described above for the backsheet. In addition, the strip may be an apertured material, such as an apertured film, or material which is otherwise gas permeable, such as a gas permeable film. The strip 51 supports an absorbent layer 52 which is attached to the body facing side 59 of the strip. In addition, an optional absorbent protective layer 53 may be applied to the absorbent layer 52 and can be coextensive with the strip 51. The absorbent layer 52 contains the superabsorbent polymer material of the present invention.

The absorbent bandage 50 of the present invention may also have a pressure sensitive adhesive 54 applied to the body-facing side 59 of the strip 51. Any pressure sensitive adhesive may be used, provided that the pressure sensitive adhesive does not irritate the skin of the user. Suitably, the pressure sensitive adhesive is a conventional pressure sensitive adhesive which is currently used on similar conventional bandages. This pressure sensitive adhesive is preferably not placed on the absorbent layer 52 or on the absorbent protective layer 53 in the area of the absorbent layer 52. If the absorbent protective layer is coextensive with the strip 51, then the adhesive may be applied to areas of the absorbent protective layer 53 where the absorbent layer 52 is not located. By having the pressure sensitive adhesive on the strip 51, the bandage is allowed to be secured to the skin of a user in need of the bandage. To protect the pressure sensitive adhesive and the absorbent, a release strip 55 can be placed on the body facing side 59 of the bandage. The release liner may be removably secured to the article attachment adhesive and serves to prevent premature contamination of the adhesive before the absorbent article is secured to, for example, the skin. The release liner may be placed on the body facing side of the bandage in a single piece (not shown) or in multiple pieces, as is shown in FIG 5A.

In another aspect of the present invention, the absorbent layer of the bandage may be placed between a folded strip. If this method is used to form the bandage, the strip is suitably fluid permeable.

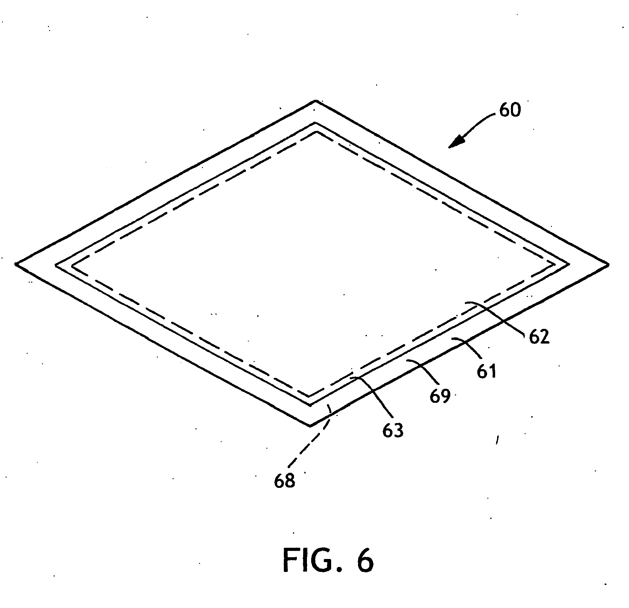

Absorbent furniture and/or bed pads or liners are also included within the present invention. As is shown in FIG 6, a furniture or bed pad or liner 60 (hereinafter referred to as a "pad") is shown in perspective. The pad 60 has a fluid impermeable backsheet 61 having a furniture-facing side or surface 68 and an upward facing side or surface 69 which is opposite the furniture-facing side or surface 68. The fluid impermeable backsheet 61 supports an absorbent layer 62 which is attached to the upward facing side 69 of the fluid impermeable backsheet. In addition, an optional absorbent protective layer 63 may be applied to the absorbent layer. The absorbent layer contains the superabsorbent polymer material of the present invention. The optional substrate layer of the absorbent layer can be the fluid impermeable layer 61 or the absorbent protective layer 63 of the pad. In the alternative, in aspects where the absorbent layer has three layers, the three layers of the absorbent layer can include the fluid impermeable layer 61, the superabsorbent polymer material layer 62 and the absorbent protective layer 63.

To hold the pad in place, the furniture-facing side 68 of the pad may contain a pressure sensitive adhesive, a high friction coating or other suitable material which will aid in keeping the pad in place during use. The pad of the present invention can be used in a wide variety of applications including placement on chairs, sofas, beds, car seats and the like to absorb any fluid which may come into contact with the pad.

The present invention may be better understood with reference to the following examples.

**TEST PROCEDURES**

**Free Swell Gel Bed Permeability Test**

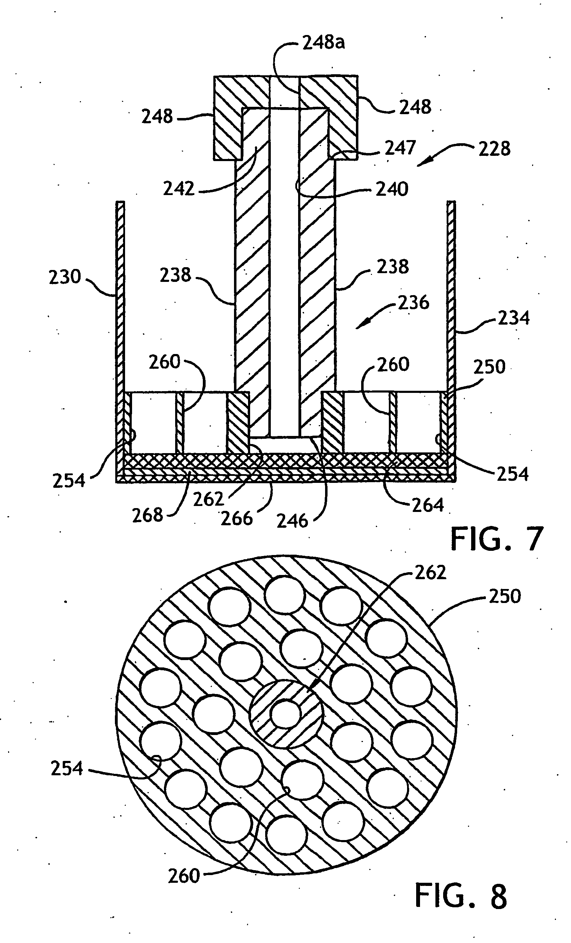

As used herein, the Free Swell Gel Bed Permeability (GBP) Test determines the permeability of a swollen bed of gel particles (e.g., such as the surface treated absorbent material or the superabsorbent material prior to being surface treated), under what is commonly referred to as "free swell" conditions. The term "free swell" means that the gel particles are allowed to swell without a restraining load upon absorbing a test solution as will be described. A suitable apparatus for conducting a Permeability Test is shown in FIGs 7 and 8 and indicated generally as 228. The test apparatus 228 comprises a sample container, generally indicated at 230, and a piston, generally indicated at 236. The piston 236 comprises a cylindrical LEXAN shaft 238 having a concentric cylindrical hole 240 bored down the longitudinal axis of the shaft. Both ends of the shaft 238 are machined to provide upper and lower ends respectively designated 242, 246. A weight, indicated as 248, rests on one end 242 and has a cylindrical hole 248a bored through at least a portion of its center.

A circular piston head 250 is positioned on the other end 246 and is provided with a concentric inner ring of seven holes 260, each having a diameter of about 0.95 cm, and a concentric outer ring of fourteen holes 254, also each having a diameter of about 0.95 cm. The holes 254, 260 are bored from the top to the bottom of the piston head 250. The piston head 250 also has a cylindrical hole 262 bored in the center thereof to receive end 246 of the shaft 238. The bottom of the piston head 250 may also be covered with a biaxially stretched 400 mesh stainless steel screen 264.

The sample container 230 comprises a cylinder 234 and a 400 mesh stainless steel cloth screen 266 that is biaxially stretched to tautness and attached to the lower end of the cylinder. A superabsorbent polymer sample, indicated as 268 in FIG 7, is supported on the screen 266 within the cylinder 234 during testing.

The cylinder 234 may be bored from a transparent LEXAN rod or equivalent material, or it may be cut from a LEXAN tubing or equivalent material, and has an inner diameter of about 6 cm (e.g., a cross sectional area of about 28.27 cm$^2$), a wall thickness of about 0.5 cm and a height of approximately 10 cm. Drainage holes (not shown) are formed in the sidewall of the cylinder 234 at a height of approximately 7.8 cm above the screen 266 to allow liquid to drain from the cylinder to thereby maintain a fluid level in the sample container at approximately 7.8 cm above screen 266. The piston head 250 is machined from a LEXAN rod or equivalent material and has a height of approximately 16 mm and a diameter sized such that it fits within the cylinder 234 with minimum wall clearance but still slides freely. The shaft 238 is machined from a LEXAN rod or equivalent material and has an outer diameter of about 2.22 cm and an inner diameter of about 0.64 cm.

The shaft upper end 242 is approximately 2.54 cm long and approximately 1.58 cm in diameter, forming an annular shoulder 247 to support the weight 248. The annular weight 248 has an inner diameter of about 1.59 cm so that it slips onto the upper end 242 of the shaft 238 and rests on the annular shoulder 247 formed thereon. The annular weight 248 can be made from stainless steel or from other suitable materials resistant to corrosion in the presence of the test solution, which are 0.9 weight percent sodium chloride solutions in distilled water. The combined weight of the piston 236 and annular weight 248 equals approximately 596 grams (g), which corresponds to a pressure applied to the absorbent structure sample 268 of about 0.3 pounds per square inch (psi), or about 20.7 grams/cm$^2$, over a sample area of about 28.27cm$^2$.

When the test solution flows through the test apparatus during testing as described below, the sample container 230 generally rests on a 16 mesh rigid stainless steel support screen (not shown). Alternatively, the sample container 230 may rest on a support ring (not shown) diametrically sized substantially the same as the cylinder 234 so that the support ring does not restrict flow from the bottom of the container.

To conduct the Gel Bed Permeability Test under "free swell" conditions, the piston 236, with the weight 248 seated thereon, is placed in an empty sample container 230 and the height from the bottom of the weight 248 to the top of the cylinder 234 is measured using a caliper of suitable gauge accurate to 0.01 mm. It is important to measure the height of each sample container 230 empty and to keep track of which piston 236 and weight 248 is used when using multiple test apparatus. The same piston 236 and weight 248 should be used for measurement when the superabsorbent polymer sample 268 is water swollen following saturation.

The sample to be tested is prepared from superabsorbent particles which are prescreened through a U.S. standard 30 mesh screen and retained on a U.S. standard 50 mesh screen. As a result, the test sample comprises particles sized in the range of about 300 to about 600 microns. The particles can be prescreened by hand or automatically. Approximately 2.0 grams of the sample is placed in the sample container 230, and the container, without the piston 236 and weight 248 therein, is then submerged in the test solution for a time period of about 60 minutes to saturate the sample and allow the sample to swell free of any restraining load.

At the end of this period, the piston 236 and weight 248 assembly is placed on the saturated sample 268 in the sample container 230 and then the sample container 230, piston 236, weight 248, and sample 268 are removed from the solution. The thickness of the saturated sample 268 is determined by again measuring the height from the bottom of the weight 248 to the top of the cylinder 234, using the same caliper or gauge used previously, provided that the zero point is unchanged from the initial height measurement. The height measurement obtained from measuring the empty sample container 230, piston 236, and weight 248 is subtracted from the height measurement obtained after saturating the sample 268. The resulting value is the thickness, or height "H" of the swollen sample.

The permeability measurement is initiated by delivering a flow of the test solution into the sample container 230 with the saturated sample 268, piston 236, and weight 248 inside. The flow rate of test solution into the container is adjusted to maintain a fluid height of about 7.8 cm above the bottom of the sample container. The quantity of solution passing through the sample 268 versus time is measured gravimetrically. Data points are collected every second for at least twenty seconds once the fluid level has been stabilized to and maintained at about 7.8 cm in height. The flow rate Q through the swollen sample 268 is determined in units of grams/second (g/g) by a linear least-square fit of fluid passing through the sample 268 (in grams) versus time (in seconds).

Permeability in Darcy is obtained by the following equation:

$$K = [Q*H*Mu]/[A*Rho*P]$$

where K = Permeability (cm$^2$), Q = flow rate (g/rate), H = height of sample (cm), Mu = liquid viscosity (poise) (approximately one centipoise for the test solution used with the Test), A = cross-sectional area for liquid flow (cm$^2$), Rho = liquid density (g/cm$^3$), for the test solution used with this Test) and P = hydrostatic pressure (dynes/cm$^2$) (normally approximately 3,923 dynes/cm$^2$). The hydrostatic pressure is calculated from the following equation:

$$P = \rho*g*h$$

where $\rho$ = liquid density (g/cm$^3$), g = gravitational acceleration, nominally 981 cm/sec$^2$, and h = fluid height (e.g., 7.8 cm for the Permeability Test described herein).

### Gel Bed Permeability Under Load Test

As used herein, the Gel Bed Permeabiltty (GBP) Under Load Test, otherwise referred to herein as GBP at 0.021 bar (0.3 psi), determines the permeability of a swollen bed of gel particles (e.g., the superabsorbent material or the absorbent material as those terms are used herein), under conditions that are commonly referred to as being "under load" conditions. The term "under load" means that swelling of the particles is restrained by a load generally consistent with normal usage loads applied to the particles, such as sitting, walking, twisting, etc. by the wearer.

More particularly, the Gel Bed Permeability Under Load Test is substantially the same as the Free Swell Gel Bed Permeability Test set forth previously with the following exception. Referring to FIGs 7 and 8, after approximately 2.0 grams of the sample is placed in the sample container 230 and spread out evenly on the bottom of the sample container, the piston 236 and weight 248 are placed on the sample within the sample container prior to the sample container (with the piston and weight therein) being submerged in the test solution (0.9% by weight NaCl saline) for a time period of about 60 minutes. As a result, a 0.021 bar (0.3 psi) restraining load is applied to the sample as the sample becomes saturated and swells.

**Centrifuge Retention Capacity Test**

The Centrifuge Retention Capacity (CRC) Test measures the ability of the superabsorbent polymer to retain liquid therein after being saturated and subjected to centrifugation under controlled conditions. The resultant retention capacity is stated as grams of liquid retained per gram weight of the sample (g/g). The sample to be tested is prepared from particles which are pre-screened through a U.S. standard 30 mesh screen and retained on a U.S. standard 50 mesh screen. As a result, the superabsorbent polymer sample comprises particles sized in the range of about 300 to about 600 microns. The particles can be pre-screeried by hand or automatically.

The retention capacity is measured by placing about 0.2 grams of the pre-screened superabsorbent polymer sample into a water-permeable bag that will contain the sample while allowing a test solution (0.9 weight percent sodium chloride in distilled water) to be freely absorbed by the sample. A heat-sealable tea bag material, such as that available from Dexter Corporation (having a place of business in Windsor Locks, Connecticut, U.S.A.) as model designation 1234T heat sealable filter paper works well for most applications. The bag is formed by folding a 12.7 cm by 7.62 cm (5-inch by 3-inch) sample of the bag material in half and heat-sealing two of the open edges to form a 6.35 cm by 7.62 cm (2.5-inch by 3-inch) rectangular pouch. The heat seals should be about 0.64 cm (0.25. inches) inside the edge of the material. After the sample is placed in the pouch, the remaining open edge of the pouch is also heat-sealed. Empty bags are also made to serve as controls. Three samples are prepared for each superabsorbent polymer to be tested.

The sealed bags are placed submerged in a pan containing the test solution at 23˚C, making sure that the bags are held down until they are completely wetted. After wetting, the samples remain in the solution for about 30 minutes, at which time they are removed from the solution and temporarily laid on a non-absorbent flat surface.

The wet bags are then placed into the basket of a suitable centrifuge capable of subjecting the samples to a g-force of about 350. One suitable centrifuge is a CLAY ADAMS DYNAC II, model #0103 having a water collection basket, a digital rpm gauge, and a machined drainage basket adapted to hold and drain the flat bag samples. Where multiple samples are centrifuged, the samples must be placed in opposing positions within the centrifuge to balance the basket when spinning. The bags (including the wet, empty bags) are centrifuged at about 1,600 rpm (e.g., to achieve a target g-force of about 350), for 3 minutes. The bags are removed and weighed, with the empty bags (controls) being weighed first, followed by the bags containing the superabsorbent polymer samples. The amount of solution retained by the superabsorbent polymer sample, taking into account the solution retained by the bag itself, is the centrifuge retention capacity (CRC) of the superabsorbent polymer, expressed as grams of fluid per gram of superabsorbent polymer. More particularly, the retention capacity is determined by the following equation:

$$\frac{[\text{sample/bag after centrifuge}] - [\text{empty bag after centrifuge}] - [\text{dry sample weight}]}{[\text{dry sample weight}]}$$

The three samples are tested and the results are averaged to determine the retention capacity (CRC) of the superabsorbent polymer material.

**Oven Shakeout Test**

The Oven Shakeout Test measures the ability of the superabsorbent polymer to affix itself to a polyolefin material. The oven shakeout test is stated "superabsorbent material shake-out", measured in "%", from the polyolefin. Materials required for this test include a 10.2 cm (4") diameter aluminum weighing pan, a polyethylene ZIPLOC baggie, superabsorbent polymer material (SAP), 0.5 inch aluminum plate, air convention oven, RETSCH VIBRO SIEVING MACHINE model number 30.403.009, a bottom pan and 12-20 mesh screen.

The aluminum plate is placed in a standard lab oven, such as a BLUE M forced air laboratory oven (available from Thermal Product Solutions, having a place of business located in Montoursville, Pennsylvania, U.S.A.) on a rack to hold the plate in a horizontal position in the central portion of the oven. The oven is preheated to 160˚C. From a polyethylene ZIPLOC baggie, a 7.62 cm (3 inch) diameter circle of polyethylene film is cut to fit the 10.2 cm (4 inch) diameter aluminum weighing pan and the combined weight of the pan and film is recorded. The weighing pan assembly is placed on top of the aluminum plate in the preheated oven and the oven door is closed. 5.0+/- 0.05 grams of the superabsorbent polymer materials are weighed into a separate sample pan. After 5 minutes, the oven door is opened and the superabsorbent polymer materials are poured on top of the polyethylene film in the weighing pan. The weighing pan is gently shaken by the edges to ensure that the superabsorbent polymer materials are spread out over as much of the film as possible. This step needs to be completed quickly to keep the oven chamber from cooling too much. The oven door is closed for another 60 seconds. The film/pan/superabsorbent assembly is removed from the oven and placed on a suitable heat-insensitive surface in the laboratory to cool for 60 seconds.

The film/pan/superabsorbent assembly is inverted to decant loose superabsorbent polymer particles that have not come into contact with polyethylene surface. The assembly is weighed and the weight is recorded as the raw pre-shake weight. The pre-shake superabsorbent weight is calculated by subtracting the initial pan/film weight from the above raw pre-shake weight. The film/pan/superabsorbent assembly is placed on the mesh screen in the RETSCH VIBRO with the superabsorbent facing down and vibrated for 1 minute with the vibration gauge indicating intensity just to the vertical mark labeled '1.' The film/pan/superabsorbent assembly is removed and the weight is recorded as the raw post-shake weight. The post-shake superabsorbent weight is calculated by subtracting the film/pan weight from the raw post-shake weight.

The percent superabsorbent lost is calculated by the following formula:

$$\text{Superabsorbent material shake-out} = 100 * \frac{(\text{pre-shake SAM wt.} - \text{post-shake SAM wt.})}{(\text{pre-shake SAM wt.})} \%$$

Minimums of three samples are tested and the results are averaged to determine the average superabsorbent material shake-out of the sample.

### Saturated Capacity Test

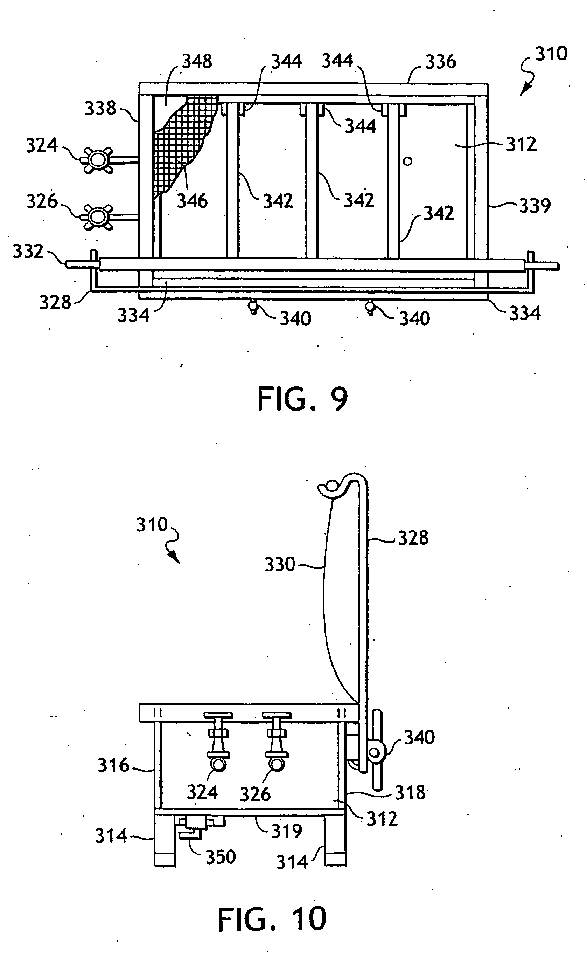

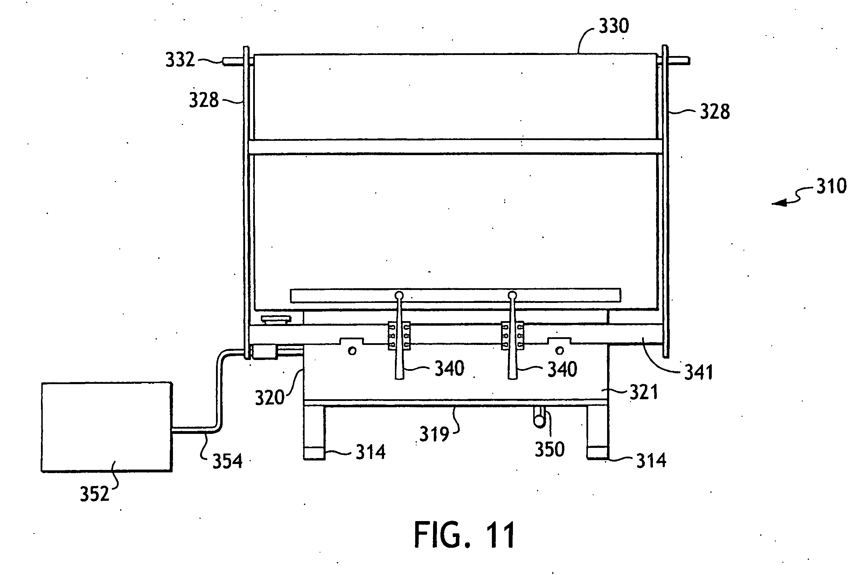

Saturated Capacity is determined using a Saturated Capacity (SAT CAP) tester with a Magnahelic vacuum gage and a latex dam, comparable to the following description. Referring to FIGs 9-11, a Saturated Capacity tester vacuum apparatus 310 comprises a vacuum chamber 312 supported on four leg members 314. The vacuum chamber 312 includes a front wall member 316, a rear wall member 318, and two side walls 320 and 321. The wall members are sufficiently thick to withstand the anticipated vacuum pressures, and are constructed and arranged to provide a chamber having outside dimensions measuring 59.7 cm (23.5 inches) in length, 35.6 cm (14 inches) in width and 20.3 cm (8 inches) in depth.

A vacuum pump (not shown) operably connects with the vacuum chamber 312 through an appropriate vacuum line conduit and a vacuum valve 324. In addition, a suitable air bleed line connects into the vacuum chamber 312 through an air bleed valve 326. A hanger assembly 328 is suitably mounted on the rear wall 318 and is configured with S-curved ends to provide a convenient resting place for supporting a latex dam sheet 330 in a convenient position away from the top of the vacuum apparatus 310. A suitable hanger assembly can be constructed from 0.64 cm (0.25 inch) diameter stainless steel rod. The latex dam sheet 330 is looped around a dowel member 332 to facilitate grasping and to allow a convenient movement and positioning of the latex dam sheet 330. In the illustrated position, the dowel member 332 is shown supported in a hanger assembly 328 to position the latex dam sheet 330 in an open position away from the top of the vacuum chamber 312.

A bottom edge of the latex dam sheet 330 is clamped against a rear edge support member 334 with suitable securing means, such as toggle clamps 340. The toggle clamps 340 are mounted on the rear wall member 318 with suitable spacers 341 which provide an appropriate orientation and alignment of the toggle clamps 340 for the desired operation. Three support shafts 342 are 1.91 cm (0.75 inches) in diameter and are removably mounted within the vacuum chamber 312 by means of support brackets 344. The support brackets 344 are generally equally spaced along the front wall member 316 and the rear wall member 318 and arranged in cooperating pairs. In addition, the support brackets 344 are constructed and arranged to suitably position the uppermost portions of the support shafts 342 flush with the top of the front, rear and side wall members of the vacuum chamber 312. Thus, the support shafts 342 are positioned substantially parallel with one another and are generally aligned with the side wall members 320 and 321. In addition to the rear edge support member 334, the vacuum apparatus 310 includes a front support member 336 and two side support members 338 and 339. Each side support member measures about 2.5 cm (1 inch) in width and about 3.2 cm (1.25 inches) in height. The lengths of the support members are constructed to suitably surround the periphery of the open top edges of the vacuum chamber 312, and are positioned to protrude above the top edges of the chamber wall members by a distance of about 1.27 cm (0.5 inches).

A layer of egg crating type material 346 is positioned on top of the support shafts 342 and the top edges of the wall members of the vacuum chamber 312. The egg crate material extends over a generally rectangular area measuring 59.7 cm (23.5 inches) by 35.6 cm (14 inches), and has a depth measurement of about 1.0 cm (0.38 inches). The individual cells of the egg crating structure measure about 0.5 inch square, and the thin sheet material comprising the egg crating is composed of a suitable material, such as polystyrene. For example, the egg crating material can be McMaster-Carr Supply Catalog No. 162 4K 14 (available from McMaster-Carr Supply Company, having a place of business in Atlanta, Georgia U.S.A.) translucent diffuser panel material. A layer of 6 mm (0.24 inch) mesh TEFLON-coated screening 348 (available from Eagle Supply and Plastics, Inc., having a place of business in Appleton, Wisconsin, U.S.A.) which measures 59.7 cm (23.5 inches) by 35.6 cm (14 inches), is placed on top of the egg crating material 346.

A suitable drain line and a drain valve 350 connect to the bottom plate member 319 of the vacuum chamber 312 to provide a convenient mechanism for draining liquids from the vacuum chamber 312. The various wall members and support members of the vacuum apparatus 310 may be composed of a suitable non-corroding, moisture-resistant material, such as polycarbonate plastic. The various assembly joints may be affixed by solvent welding and/or fasteners, and the finished assembly of the tester is constructed to be water-tight. A vacuum gauge 352 operably connects through a conduit into the vacuum chamber 312. A suitable pressure gauge is a Magnahelic differential gauge capable of measuring a vacuum of 0.254 cm (0-100 inches) of water, such as a No. 2100 gauge available from Dwyer Instrument Incorporated (having a place of business in Michigan City, Indiana, U.S.A.)

The dry product or other absorbent structure is weighed and then placed in excess 0.9% NaCl saline solution, submerged and allowed to soak for twenty (20) minutes. After the twenty (20) minute soak time, the absorbent structure is placed on the egg crate material and mesh TEFLON-coated screening of the Saturated Capacity tester vacuum apparatus 310. The latex dam sheet 330 is placed over the absorbent structure(s) and the entire egg crate grid so that the latex dam sheet 330 creates a seal when a vacuum is drawn on the vacuum apparatus 310. A vacuum of 0.034 bar (5 pounds per square inch (psi)) is held in the Saturated Capacity tester vacuum apparatus 310 for five minutes. The vacuum creates a pressure on the absorbent structure(s), causing drainage of some liquid. After five minutes at 0.034 bar (0.5 psi) vacuum, the latex dam sheet 330 is rolled back and the absorbent structure(s) are weighed to generate a wet weight.

The overall capacity of each absorbent structure is determined by subtracting the dry weight of each absorbent from the wet weight of that absorbent, determined at this point in the procedure. The 0.034 bar (0.5 psi) Saturated Capacity or Saturated Capacity of the absorbent structure is determined by the following formula:

$$\text{Saturated Capacity} = (\text{wet weight} - \text{dry weight})/ \text{dry weight};$$

wherein the Saturated Capacity value has units of grams of fluid/gram of absorbent. For Saturated Capacity, a minimum of three specimens of each sample should be tested and the results averaged. If the absorbent structure has low integrity or disintegrates during the soak or transfer procedures, the absorbent structure can be wrapped in a containment material such as paper toweling, for example SCOTT paper towels manufactured by Kimberly-Clark Corporation, having a place of business in Neenah, Wisconsin, U.S.A. The absorbent structure can be tested with the overwrap in place and the capacity of the overwrap can be independently determined and subtracted from the wet weight of the total wrapped absorbent structure to obtain the wet absorbent weight.

**Fluid Intake Rate Test**

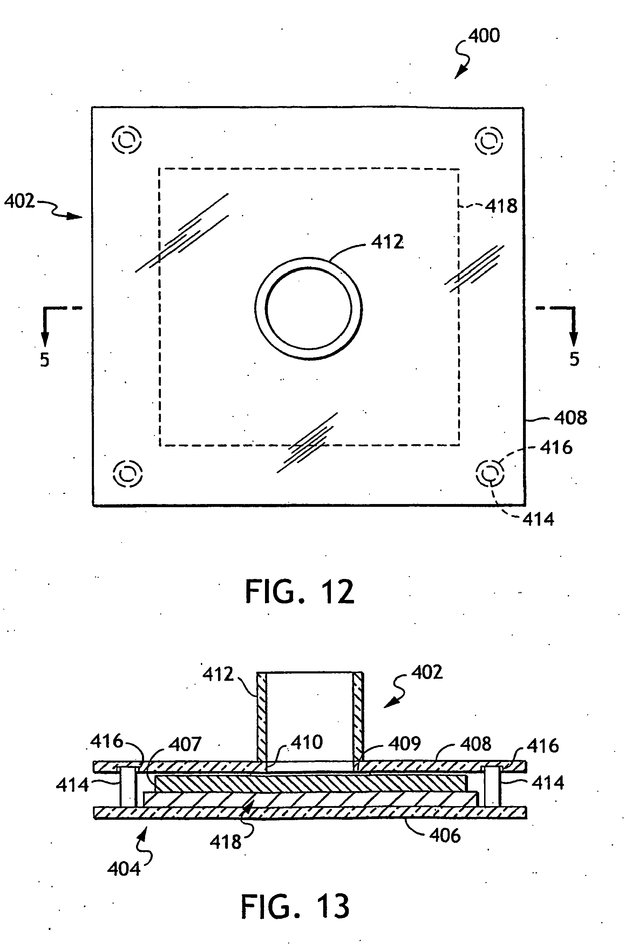

The Fluid Intake Rate (FIR) Test determines the amount of time required for an absorbent structure to take in (but not necessarily absorb) a known amount of test solution (0.9 weight percent solution of sodium chloride in distilled water at room temperature). A suitable apparatus for performing the FIR Test is shown in FIGs 12 and 13 and is generally indicated at 400. The test apparatus 400 comprises upper and lower assemblies, generally indicated at 402 and 404 respectively, wherein the lower assembly comprises a generally 7 inch by 7 inch (17.8 cm x 17.8 cm) square lower plate 406 constructed of a transparent material such as PLEXIGLAS (available from Degussa AG, having a place of business in Dusseldorf, Germany) for supporting the absorbent sample during the test and a generally 11.4 cm x 11.4 cm (4.5 inch by 4.5 inch) square platform 418 centered on the lower plate 406.

The upper assembly 402 comprises a generally square upper plate 408 constructed similar to the lower plate 406 and having a central opening 410 formed therein. A cylinder (fluid delivery tube) 412 having an inner diameter of about 2.5 cm (one inch) is secured to the upper plate 408 at the central opening 410 and extends upward substantially perpendicular to the upper plate. The central opening 410 of the upper plate 408 should have a diameter at least equal to the inner diameter of the cylinder 412 where the cylinder 412 is mounted on top of the upper plate 408. However, the diameter of the central opening 410 may instead be sized large enough to receive the outer diameter of the cylinder 412 within the opening so that the cylinder 412 is secured to the upper plate 408 within the central opening 410.

Pin elements 414 are located near the outside corners of the lower plate 406, and corresponding recesses 416 in the upper plate 408 are sized to receive the pin elements 414 to properly align and position the upper assembly 402 on the lower assembly 404 during testing. The weight of the upper assembly 402 (e.g., the upper plate 408 and cylinder 412) is approximately 360 grams to simulate approximately 0.0076 bar (0.11 pounds/square inch (psi)) pressure on the absorbent sample during the FIR Test.

To run the FIR Test, an absorbent sample 407 being three inches (7.6 cm) in diameter is weighed and the weight is recorded in grams. The sample 407 is then centered on the platform 418 of the lower assembly 404. The upper assembly 402 is placed over the sample 407 in opposed relationship with the lower assembly 404, with the pin elements 414 of the lower plate 406 seated in the recesses 416 formed in the upper plate 408 and the cylinder 412 is generally centered over the sample 407. Prior to running the FIR test, the aforementioned Saturated Capacity Test is measured on the sample 407. Thirty percent (30%) of the saturation capacity is then calculated by multiplying the mass of the dry sample (grams) times the measured saturated capacity (gram/gram) times 0.3; e.g., if the test sample has a saturated capacity of 20g of 0.9% NaCl saline test solution/g of test sample and the 7.6 cm (three inch) diameter sample 407 weighs one gram, then 6 grams of 0.9% NaCl saline test solution (referred to herein as a first insult) is poured into the top of the cylinder 412 and allowed to flow down into the absorbent sample 407. A stopwatch is started when the first drop of solution contacts the sample 407 and is stopped when the liquid ring between the edge of the cylinder 412 and the sample 407 disappears. The reading on the stopwatch is recorded to two decimal places and represents the intake time (in seconds) required for the first insult to be taken into the absorbent sample 407.

A time period of fifteen minutes is allowed to elapse, after which a second insult equal to the first insult is poured into the top of the cylinder 412 and again the intake time is measured as described above. After fifteen minutes, the procedure is repeated for a third insult. An intake rate (in milliliters/second) for each of the three insults is determined by dividing the amount of solution (e.g., six grams) used for each insult by the intake time measured for the corresponding insult.

At least three samples of each absorbent test are subjected to the FIR Test and the results are averaged to determine the intake rate.

### Composite Stretchability Test

To determine the stretchability of a material, a predetermined amount of elongation is chosen: e.g. 30%, 50%, 100% etc. The amount of stretch provides a corresponding stretch ratio: e.g. 130%, 150% 200% etc. For each level of stretchability testing, three specimens are tested and all three must pass in order to consider the sample as having the respective level of stretchability. A sufficient amount of stretching force is applied to the specimen, as needed to generate the selected amount of stretching. Each specimen is tested only once, even if the specimen is not damaged.

The stretchability of a composite is measured after 3 cycles with each cycle including (a) stretching to a predetermined ratio of extension, and (b) releasing the stretching force, thereby allowing the stretched composite to retract back toward its original dimension.

Stretchability is defined according to the following equation:

$$\text{Stretchability} = (L_e - L_o) \times 100\% / L_o$$

wherein $L_e$ is the length after extension (i.e., at the predetermined ratio), and $L_o$ is the original sample length. For a sample to be qualified as having a predetermined stretchability, the sample must be able to demonstrate all of the following requirements:

The sample must be able to reach the predetermined stretch ratio.

The sample must be able to retract at least 70% of the extension within a 1-minute interval after the applied stretching force is removed, as measured in the third stretching cycle. The percent retraction is defined as:

$$\text{Retraction (\%)} = \{1 - (L_f - L_o) / (L_e - L_o)\} \times 100\%$$

wherein $L_f$ is the sample length after the force has been released for 1-minute, $L_e$ is the length after extension (i.e., the predetermined ratio), and $L_o$ is the original sample length before extension.

The sample must meet the first criterion after the first and second extension, and meet the second criterion after the third extension on the same specimen. (In carrying out the test, the retraction criterion is measured only after the third extension).

Desirably, the sample exhibits no visible structural defects, such as excessive voids or cracks, after the stretchability testing.

The absorbent composite is cut into 7.62 cm (3-inch) by 17.78 cm (7-inch) specimens. An INSTRON 4443 (available from Instron Corporation, having a place of business in Canton, Massachusetts, U.S.A.) is used to measure stretchability. A substantially equivalent testing device may optionally be employed. Each specimen is mounted onto the equipment vertically with two clamps and the locations of the clamps are marked on the specimen. The distance between the two clamps ($L_o$) is 10.16 cm (4 inches). The specimen is stretched by moving the upper clamp upward at a rate of 500 mm/min and is held for 5 seconds at the predetermined length of extension ($L_e$). After 5 seconds of holding, the upper clamp is returned to the original position and the specimen is free to retract. The second cycle of stretching is started after the upper clamp is back in the original position for 10 seconds, followed by the third cycle. The stretching and retraction procedure for the second and third cycles is the same as the first cycle.

The specimen is removed from the equipment after completion of the third stretching cycle and is laid on the bench. The distance between the two marks ($L_f$) is measured after the specimen is relaxed for 1 minute. For each absorbent composite, test specimens are prepared and subjected to stretchability testing with respect to both the machine direction (MD) and the cross-machine direction (CD) of the absorbent composite. The lower stretchability value measured from the CD and MD directions is chosen to represent stretchability of the absorbent composite.

### Composite Shakeout Test

A suitable apparatus and procedure for determining the composite shake-out value of a sample material is described in PCT publication WO 02/076520 entitled HIGH SUPERABSORBENT CONTENT WEBS AND A METHOD FOR MAKING THEM published 10/3/2002.

The susceptibility of a superabsorbent/fiber web to the migration and escape of superabsorbent material (SAM) can be measured by employing a composite shakeout test procedure which involves agitating web samples in a controlled fashion and determining the total loss of web mass from the sample. A sample of the stretchable absorbent composite is prepared in the shape of a rectangular plate which has a length of 22.86 cm (9 inches) and a width of 10.16 cm (4 inches). The sample has the density that would be present when the absorbent composite is incorporated in its intended end-product article. Any tissues or other layers that were employed during the process of forming the sample materials are removed from all specimens before conducting the Composite Shakeout Test.

The Composite Shakeout Test can be conducted by employing a Model # RX-24 PORTABLE SIEVE SHAKER (herein after referred to as "RX-24") available from W.S. Tyler Inc. (having a place of business in Mentor, Ohio, U.S.A.). The shaker apparatus is modified in the manner described in PCT publication WO 02/076520, which corresponds to U.S. Patent Application Publication 2002/0183703 A1. For use in the Composite Shakeout Tests, the RX-24 is modified to shake web samples and allow a determination of the web's resistance to the migration of superabsorbent material (SAM), based on the mass of web material lost during the shaking. The modifications to the shaker apparatus involve making changes to the guide frame in the manner described in PCT publication WO 02/076520. In addition to the changes to the guide frame described in this PCT publication, a modified sample holder is employed in the shakeout test. The sample holder has a frame made of polyacrylate plate and two pieces of mesh screen. The frame has a length of 43.18 cm (17 inches), a width of 29.21 cm (11.5 inches) and a thickness of 0.51 cm (0.20 inch). The frame has a rectangular opening with a length of 38.74 cm (15.25 inches) and a width of 15.88 cm (6.25 inches), and the opening is substantially centered in the frame. One piece of mesh screen with a dimension slightly larger than the opening is operatively joined on each side of the frame (e.g., with duct tape) to hold the test sample. The mesh screen has 0.4 cm x 0.4 cm square openings, and the total weight of the sample holder is about 500 grams. A substantially equivalent shaker system may optionally be employed.

To perform the Composite Shakeout Test, the absorbent composite sample is laid at the center of the sample holder, and the sample holder is laid horizontally flat (i.e. parallel to the floor) upon the wire screen employed to support the sample on the modified RX-24. The RX-24 then shakes the web at a frequency of 520 cycles per minute for a period of five minutes. If any sheets of tissue paper or other material have been placed above or below the sample to facilitate the lifting or handling of the web samples, those sheets are removed prior to shaking.

After the completion of the shaking portion of the test, the mass loss and the superabsorbent-loss are determined by comparing the total remaining mass of the absorbent composite sample with the original mass of the sample when the sample was initially placed on the support screen, in accordance with the following formula:

$$\text{Mass loss (\%)} = 100\% \times ((M_0 - M_{end}) \div M_0)$$

where: $M_0$ = sample mass prior to shakeout test (e.g. grams);

$M_{end}$ = sample mass remaining after test (e.g. grams).

Mass that is lost from the sample will generally fall through the openings in the support screen. Any mass that remains on the screen is counted as mass loss.

The superabsorbent shakeout value (%) is the total mass loss (%) produced at the above-described shaking conditions.

While the foregoing discussion has described in detail one desirable method for conducting the Composite Shakeout test using a specific type of apparatus, it will be appreciated that those skilled in the art will be able to prepare other apparatus that will allow equivalent testing in which agitation applied to webs will yield the identical results in terms of web loss as that achieved by the disclosed Composite Shakeout Test. Accordingly, the scope of the Composite Shakeout Test will include any equivalent test methods for determining web loss.

### Composite Permeability Test

The Composite Permeability Test is used to determine the permeability of the absorbent composite, and more particularly a "z-direction" permeability of the absorbent composite based on liquid flow through the thickness of the composite. This test is substantially similar to the Gel Bed Permeability Test set forth above, with the following noted exceptions. Referring back to FIGs 7 and 8, instead of being swelled under a 0.021 bar (0.3 psi) pressure, the absorbent composites were swelled under no load but tested their permeability under a 0.021 bar (0.3 psi) pressure (the piston 236 and weight 248 were placed onto the absorbent composite after it was freely saturated for 60 minutes in a 0.9 weight percent NaCl saline solution). Also, instead of particulate superabsorbent material being placed in the sample container 230, a circular absorbent composite sample 268 (e.g., either formed or otherwise cut from a larger absorbent composite), with any forming material wrap (e.g., forming tissue) removed and having a diameter of about 6 cm is placed in the sample container 230 at the bottom of the cylinder 234 in contact with the screen 264. The sample container 230 (without the piston and weight therein) is then submerged in a 0.9 weight percent NaCl saline solution for a time period of about 60 minutes to saturate the absorbent composite. The same height measurement obtained for the GBP Test are taken, e.g., with the sample container 230 empty and with the absorbent composite sample within the sample container 230 and saturated.

The absorbent composite permeability measurement is initiated by delivering a continuous flow of saline solution into the sample container 230 with the saturated absorbent composite, the piston 236, and the weight 248 inside. The saline solution is delivered to the sample container 230 at a flow rate sufficient to maintain a fluid height of about 7.8 cm above the bottom of the sample container 230. The quantity of fluid passing through the absorbent composite versus time is measured gravimetrically. Data points are collected every second for at least twenty seconds once the fluid level has been stabilized to and maintained at about 7.8 cm in height. The flow rate Q through the absorbent composite sample 268 is determined in units of grams/second (g/s) by a linear least-square fit of fluid passing through the sample container 230 (in grams) versus time (in seconds). The permeability of the absorbent composite is then determined using the equation set forth above for the Gel Bed Permeability Test.

Where the Composite Permeability Test is conducted as described above, and more particularly where the absorbent composite sample is submerged in the solution without the piston and weight thereon, the test is said to be conducted under "free swell" conditions whereby the absorbent composite is allowed to swell free of any restraining load. In a variation of this test, the piston and weight may be placed on the sample within the sample container 230 and then the entire assembly can be submerged so that a load is applied to the sample as the sample becomes saturated and swells. When conducted in this manner the test is referred to as being conducted "under load."

### EXAMPLES

The following examples are provided to illustrate the invention, and do not limit the scope of the claims. Unless otherwise stated all parts and percentages are by weight.

### Preproduct

In an insulated, flat-bottomed reaction vessel, 1866.7 g of 50% NaOH was added to 3090.26 g of distilled water and cooled to 25 ˚C. 800 g of acrylic acid was then added to the caustic solution and the solution was again cooled to 25 ˚C. A second solution of 1600 g of acrylic acid containing 120 g of 50% by weight methoxypolyethyleneglycol monomethacrylate in acrylic acid and 14.4 g of ethoxylated trimethylolpropanetriacrylate were then added to the first solution, followed by cooling to 15 ˚C, the addition of 14.4 g of hydroxymonoallyl ether with 10 moles of ethoxylation, and additional cooling to 5 ˚C, all while stirring. The monomer solution was then polymerized with a mixture of 100 ppm hydrogen peroxide, 200 ppm azo-bis-(2-amidino-propene)dihydrochloride, 200 ppm sodiumpersulfate and 40 ppm ascorbic acid (all aqueous solutions) under adiabatic conditions and held near the maximum temperature for 25 minutes. The resulting gel was chopped and extruded with a HOBART 4M6 commercial extruder, followed by drying in a Procter & Schwartz Model 062 forced air oven at 175 ˚C for 10 minutes with up flow and 6 minutes with down flow air on a 20in X 40in perforated metal tray to a final product moisture level of less than 5 wt%. The dried material was coarse ground in a Prodeva Model 315-S crusher, milled in an MPI 666-F three stage roller mill and sieved with an Minox MTS 600DS3V to remove particles greater than 850 microns and smaller than 150 microns.

## Comparative Example 1 and Examples 1-3

In accordance with Table 1 for Comparative Examples 1 and Examples 1 to 3 3,000g of the Preproduct were blended at ambient conditions with amounts of a 25% emulsion of maleated polypropylene (MPP) as shown in Table 1. In particular, the Preproduct was fluidized. A spray solution was prepared containing ethylene carbonate (EC), thermoplastic coating and water in accordance with the amounts given in Table 1. The spray solution was sprayed onto the fluidized Preproduct. The spray blend was formulated to deliver 1% ethylene carbonate, 0.0625% MPP, and 4% water, with the amount of water calculated to include what the MPP emulsion contributed on top of the added water. Then, 24.0 grams of fumed silica was added to the sprayed Preproduct. The total mixture was fluidized for about 1 minute. EC/thermoplastic/water coated preproduct was fed into a continuous paddle dryer for 50 minutes at 60-70 grams/minute with a 2.5-3kg steady-state polymer mass in the reactor. The peak superabsorbent temperature was reached near the middle of the second half of the reactor, and this temperature was held to 190-195˚C. Paddles were held to 25 rpm. The material was then further treated with an aqueous solution of PEG8000.

For each Comparative Example 1 and Examples 1-3, samples were tested by the Oven Shakeout Procedure as set forth herein. The superabsorbent material shakeout of the Comparative Example 1 and Examples 1-3 were measured and are found in Table 2. It can be seen from Table 2, that superabsorbent polymer material of Examples 1 to 3 have a lower superabsorbent material shakeout than other superabsorbent polymer material of the comparative examples.

### Table 1 - Comparative Example 1 and Examples 1-3

| Comparative Example and Examples | Ethylene Carbonate % on SAP | Thermoplastic Coating % on SAP | Water % on SAP | Silica % | CRC g/g | GBP 0 bar (0.0psi) Darcy | GBP 0.021 bar (0.3psi) Darcy |
|---|---|---|---|---|---|---|---|
| Comp Ex 1 | 1 | none | 4 | 0 | 25.9 | 37 | 10.23 |
| Example 1 | 1 | 0.0625 MPP[1] | 4 | 0 | 26 | 111 | 7.65 |
| Example 2 | 1 | 0.0625 MPP[1] | 4 | 0.4 | 25.7 | 152 | 16.04 |
| Example 3 | 1 | 0.0625 MPP[1] | 4 | 0.8 | 25.5 | 156 | 11.29 |
| MPP[1] maleated polypropylene | | | | | | | |

### Table 2 - Superabsorbent Material (SAM) Shakeout Results for Comparative Example 1 and Examples 1-3

| Comparative Examples and Examples | pan + film (g) | preshake (g) | post shake (g) | % SAM retained | SAM Shakeout | SAM Shakeout Average |
|---|---|---|---|---|---|---|
| Comparative Example 1a | 2.8381 | 4.1056 | 3.4654 | 49.49 | 50.51 | |
| Comparative Example 1b | 2.8556 | 3.7338 | 3.4738 | 70.39 | 29.61 | 41.51 |
| Comparative Example 1c | 2.8692 | 4.1281 | 3.569 | 55.59 | 44.41 | |
| Example 1a | 2.8306 | 3.4794 | 3.3824 | 85.05 | 14.95 | |
| Example 1b | 2.8203 | 3.5562 | 3.4829 | 90.04 | 9.96 | 12.88 |
| Example 1c | 2.8604 | 3.8075 | 3.6774 | 86.26 | 13.74 | |
| Example 2a | 2.8441 | 3.4821 | 3.3853 | 84.83 | 15.17 | |

(continued)

| Comparative Examples and Examples | pan + film (g) | preshake (g) | post shake (g) | % SAM retained | SAM Shakeout | SAM Shakeout Average |
|---|---|---|---|---|---|---|
| Example 2b | 2.8412 | 3.2991 | 3.2691 | 93.45 | 6.55 | 7.96 |
| Example 2c | 2.8404 | 3.2888 | 3.2468 | 90.63 | 9.37 | |
| Example 3a | 2.8488 | 3.7219 | 3.6421 | 90.86 | 9.14 | |
| Example 3b | 2.8357 | 3.3495 | 3.2912 | 88.65 | 11.35 | 10.32 |
| Example 3c | 2.857 | 3.8653 | 3.7598 | 89.54 | 10.46 | |

## Examples 4-5 (according to the invention)

In accordance with Table 3 for Example 4 and Example 5, 3,000g of the Preproduct of Example 1 was blended at ambient conditions with amounts of a 25% aqueous emulsion of a blend of maleated polypropylene and ethylene acrylic acid copolymer. In particular, the Preproduct of Example 1 was fluidized. A spray solution was prepared by dissolving ethylene carbonate in warm water, adding the maleated polypropylene to the solution than adding the ethylene acrylic acid copolymer to the solution in accordance with the amounts given in Table 3. The spray solution was sprayed onto the fluidized Preproduct. Then, 24.0 grams of fumed silica was added to the sprayed Preproduct. The total mixture was fluidized for about 1 minute. Ethylene carbonate/thermoplastic/water-coated preproduct was fed into a continuous paddle dryer for 50 minutes at 60-70 grams/minute with a 2.5-3kg steady-state polymer mass in the reactor. The peak superabsorbent temperature was reached near the middle of the second half of the reactor, and this temperature was held to 190-195°C. Paddles were held to 25 rpm.

After surface-crosslinking using ethylene carbonate and heating, the surface treated superabsorbent material was sprayed with a 1.25% solution of polyvinylamine to result in 0.25% polyvinylamine on superabsorbent by weight. To apply the solution, surface-crosslinked superabsorbent was fluidized and the liquid was sprayed onto the moving powder bed.

### Table 3 - Examples 4-5

| Examples | Ethylene Carbonate % of SAP | Thermoplastic Coating % on SAP | Water | Silica % | CRC g/g | GBP 0 bar (0.0psi) Darcy |
|---|---|---|---|---|---|---|
| Example 4 | 1 | 0.0312% MPP[1] 0.0312% EEA[2] | 114.375 | 0 | 25 | 174 |
| Example 5 | 1 | 0.0312% MPP[1] 0.0312% EEA[2] | 114.375 | 0.8 | 25.5 | 198 |
| MPP[1] = maleated polypropylene EEA[2] ethylene arcylic acid copolymer | | | | | | |

For Examples 4-5, samples were tested by the Oven Shake-Out Procedure as set forth herein. The superabsorbent material shakeout of the Examples 4-5 were measured and are found in Table 4.

### Table 4 - Superabsorbent Material (SAM) Shakeout Results for Examples 4-5

| Examples | pan + film (g) | preshake (g) | post shake (g) | % SAM retained | SAM Shakeout % |
|---|---|---|---|---|---|
| Example 4a | 2.7971 | 3.6284 | 3.5854 | 94.83 | 5.17 |
| Example 4b | 2.8346 | 4.03 | 3.8011 | 80.85 | 19.15 |
| Example 5 | 2.8251 | 3.6555 | 3.55 | 87.30 | 12.70 |

**Examples 6 - 12:**

These sample stretchable absorbent cores were prepared in a pilot coform machine and had a composite width (i.e., in the CD direction) of approximately 13.0 cm (5.5 inches). Three superabsorbents, FAVOR SXM 9394 (commercially available from Stockhausen Inc., having a place of business in Greensboro, North Carolina, U.S.A.), the superabsorbent material of Example 3 as described above and the superabsorbent material of Example 5 as described above, were chosen to incorporate into the stretchable absorbent cores, together with a polypropylene-based thermoplastic elastic polymer with a melt flow rate (MFR) of approximately 80 (available from producers such as ExxonMobil Chemical Company) and a wood pulp fiber NF 405 commercially available from Weyerhaeuser Company. The composition and process conditions for Examples 6 - 12 are listed in Table 5 and Table 6.

**Table 5 - Core Composition**

| Example | Composite Composition | | | Process Condition | |
| --- | --- | --- | --- | --- | --- |
| | Superabsorbent | Elastomer - MFR 80 Polypropylene | Wood Pulp NF405 | Line Speed (diaper/min) | Basis Weight (g/m$^2$) |
| 6 | 75% SXM 9394 | 15% | 10% | 100 | 695 |
| 7 | 75% Example 3 | 15% | 10% | 100 | 695 |
| 8 | 75% Example 3 | 15% | 10% | 175 | 695 |
| 9 | 75% Example 5 | 15% | 10% | 100 | 695 |
| 10 | 75% Example 5 | 15% | 10% | 175 | 695 |
| 11 | 79% Example 5 | 16% | 5% | 100 | 660 |
| 12 | 83% Example 5 | 17% | 0% | 100 | 623 |

**Table 6 - Process Conditions**

| Parameter | Value |
| --- | --- |
| Die | |
| Hole diameter | 0.051 cm (0.02 inch) |
| Die holes | 9 per cm (24 per inches) |
| Die air gap | 0.12 cm (0.046 inch) |
| Number of Die | 2 |
| Extruder | |
| Screw speed | 5 to 35 rpm |
| Melt pressure before filter | 0.68g to 20.7 bar (10 to 300 psig) |
| Melt pressure after filter | 0.68g to 6.8g bar (10 to 100 psig) |
| Zone 1 Temp. | 149 ˚C (300 ˚F) |
| Zone 2 Temp. | 163 ˚C (325 ˚F) |
| Zone 3 Temp. | 177 ˚C (350 ˚F) |
| Zone 4 Temp. | 204 ˚C (400 ˚F) |
| Zone 5 Temp. | 204 ˚C (400 ˚F) |
| Zone 6 Temp. | 204 ˚C (400 ˚F) |
| Clamp Temp. | 204 ˚C (400 ˚F) |
| Alux Temp. | 204 ˚C (400 ˚F) |
| Forming | |

(continued)

| Parameter | Value |
|---|---|
| Pulp chute angle | 28 degrees |
| Die 1-2 tip-to-tip distance | 14 cm (5.5 inches) |
| Die 1 or 2 to screen height | 35.6 to 45.7 cm (14 to 18 inches) |
| Die 1 or 2 tip angle | 57 degree |
| Pulp chute to screen height | 39.4 cm (15.5 inches) |
| Line | |
| Line speed | 30.5 to 61 m/min (100 to 200 feet/min) |
| Forming wire downstream surface temp. | 29.4 ˚C (~ 85 ˚F) |
| Forming wire return surface temp. | 37.8 ˚C (~100 ˚F) |
| Polymer | |
| Polymer Zone 1 melting temp. | 204 ˚C (400 ˚F) |
| Polymer output rate | 90.7 kg/h (200 lb/h) for VISTAMAXX 2370 & 27.2 kg/h (60 lb/h) for polypropylene with 80 MFR |
| Primary Air | |
| Furnace blow off pressure | 0.55 to 0.6g bar (8 to 10 psig) |
| Die tip 1 or 2 valve pressure Die tip 1 or 2 valve temp. | 0.41 to 0.55 bar (6 to 8 248˚C (478 ˚F) for VISTAMAXX 2370 & 274˚C (525 ˚F) for polypropylene with 80 MFR |
| Forming drum air pressure | 76 cm (30 inches) $H_2O$ |
| Die tip air flow | 140 SCFM |
| Pulp | |
| Pulp feed roll speed | 7.62 to 9.65 m/minute (300 to 380 inch/minute) |
| Pulp transport air pressure | ~ 0.25 cm (~ 0.1 inch) $H_2O$ |
| SAM | |
| SAM feed screw speed | ~ 200 rpm |
| SAM transport air pressure | ~ 6.35 cm (~ 2.5 inches) $H_2O$ |

The resulting absorbent cores were then tested for superabsorbent material (SAM) shakeout using the Composite Shakeout Test, for absorbency

(saturated capacity, or "sat. cap.") using the Saturated Capacity Test, for permeability using the Composite Permeability Test, and for fluid intake rate using the Fluid Intake Rate Test. The results of these tests can be seen in Table 7.

**Table 7 - Core Absorbent Properties**

| Example | Shakeout (%) | Sat. Cap. (g/g) | Permeability (darcy) | | Fluid Intake Rate (ml/sec) | | | |
|---|---|---|---|---|---|---|---|---|
| | | | 0 bar (0 psi) | 0.021 bar (0.3 psi) | 1st | 2nd | 3rd | 4th |
| 6 | 4.3 | 19.4 | 9.26 | 1.20 | 0.818 | 0.599 | 0.429 | 0.431 |
| 7 | 2.0 | 20.2 | 41.12 | 4.30 | 0.764 | 1.399 | 1.159 | 0.949 |
| 8 | 9.7 | 20.0 | 64.99 | 13.91 | 0.856 | 1.598 | 2.166 | 1.468 |
| 9 | 2.0 | 20.7 | 41.28 | 4.75 | 0.797 | 1.698 | 2.046 | 1.660 |
| 10 | 3.0 | 20.6 | 42.36 | 4.57 | 0.991 | 1.763 | 2.306 | 1.875 |

(continued)

| Example | Shakeout (%) | Sat. Cap. (g/g) | Permeability (darcy) | | Fluid Intake Rate (ml/sec) | | | |
|---|---|---|---|---|---|---|---|---|
| | | | 0 bar (0 psi) | 0.021 bar (0.3 psi) | 1st | 2nd | 3rd | 4th |
| 11 | 3.1 | 18.8 | 35.47 | 1.83 | 0.625 | 1.365 | 2.161 | 2.256 |
| 12 | 2.6 | 16.4 | 78.91 | 1.28 | 0.638 | 0.613 | 1.261 | 1.202 |

Table 7 demonstrates that the absorbent cores of the present disclosure exhibit significantly improved absorbent properties when compared to absorbent cores utilizing conventional superabsorbent polymer materials.

### Examples 13 - 19:

The stretchable absorbent cores for these examples were prepared using the pilot coform machine described above. These example absorbent core likewise had a composite width (i.e., in the CD direction) of approximately 13.0 cm (5.5 inches). Further improvement in balancing superabsorbent attachment and fluid handling properties was accomplished by using a different grade of elastomeric polymer and varied amounts of wood pulp fiber. The thermoplastic elastomeric polymer for these examples was VISTAMAXX 2370 (commercially available from ExxonMobil Chemical Company). The process conditions can be seen in Table 6 above and the absorbent core compositions can be seen in Table 8 below. The resulting absorbent cores were then tested for superabsorbent material (SAM) shakeout using the Composite Shakeout Test, for absorbency (saturated capacity, or "sat. cap.") using the Saturated Capacity Test, for permeability using the Composite Permeability Test, and for fluid intake rate using the Fluid Intake Rate Test. The results of these tests can be seen in Table 9.

**Table 8 - Core Compositions**

| Example | Composite Composition | | | Process Condition | |
|---|---|---|---|---|---|
| | Superabsorbent | Elastomer-VISTAMAXX 2370 | Wood Pulp NF405 | Line Speed (diaper/min) | Basis Weight (g/m$^2$) |
| 13 | 75% SXM9394 | 15% | 10% | 100 | 700 |
| 14 | 75% Example 3 | 15% | 10% | 100 | 700 |
| 15 | 72% Example 3 | 14% | 10% | 100 | 700 |
| 16 | 68% Example 3 | 14% | 10% | 100 | 700 |
| 17 | 75% Example 5 | 15% | 10% | 100 | 700 |
| 18 | 72% Example 5 | 14% | 5% | 100 | 700 |
| 19 | 68% Example 5 | 14% | 0% | 100 | 700 |

**Table 9 - Core Absorbent Properties**

| Example | Shake out (%) | Sat. Cap. (g/g) | Permeability (Darcy) | | Fluid Intake Rate (ml/sec) | | | |
|---|---|---|---|---|---|---|---|---|
| | | | 0 bar (0 psi) | 0.021 bar (0.3 psi) | 1st | 2nd | 3rd | 4th |
| 13 | 2.38 | 19.2 | 10.57 | 2.17 | 0.552 | 0.600 | 0.652 | 0.577 |
| 14 | 1.43 | 17.9 | 19.2 | 1.2 | 0.565 | 0.845 | 0.935 | 0.709 |
| 15 | 0.65 | 18.8 | 18.8 | 3.6 | 0.642 | 1.199 | 1.261 | 0.742 |
| 16 | 1.07 | 20.0 | 28.3 | 9.7 | 0.971 | 1.525 | 1.266 | 0.732 |
| 17 | 0.90 | 15.5 | 66.0 | 1.9 | 0.577 | 0.757 | 1.394 | 1.700 |
| 18 | 0.71 | 18.7 | 26.9 | 3.3 | 0.909 | 1.024 | 1.446 | 1.175 |

(continued)

| Example | Shake out (%) | Sat. Cap. (g/g) | Permeability (Darcy) | | Fluid Intake Rate (ml/sec) | | | |
|---|---|---|---|---|---|---|---|---|
| | | | 0 bar (0 psi) | 0.021 bar (0.3 psi) | 1st | 2nd | 3rd | 4th |
| 19 | 1.44 | 18.7 | 29.9 | 6.9 | 1.180 | 1.241 | 1.338 | 0.920 |

It can be seen that the absorbent cores utilizing VISTAMAXX 2370 exhibit an improved superabsorbent attachment (i.e., a lower shake-out value) while exhibiting the same or improved absorbent properties, as compared to stretchable absorbent cores comprising conventional superabsorbent materials. Also, it can be seen that wood pulp fiber content affects both shake-out value and fluid handling properties.

It will be appreciated that details of the foregoing examples, given for purposes of illustration, are not to be construed as limiting the scope of this invention. Although only a few exemplary embodiments of this invention have' been described in detail above, those skilled in the art will readily appreciate that many modifications are possible in the examples without materially departing from the novel teachings and advantages of this invention. For example, features described in relation to one example may be incorporated into any other example of the invention.

**Claims**

1. An absorbent article comprising:

   a topsheet;
   a backsheet; and
   an absorbent core disposed between the topsheet and the backsheet;
   wherein the absorbent core comprises a superabsorbent polymer material comprising:

   a) from 55 to 99.9% by weight of polymerizable unsaturated acid group containing monomer based on the superabsorbent polymer, and
   b) from 0.001 to 5% by weight of internal crosslinking agent based on the polymerizable unsaturated acid group containing monomer;

   wherein the superabsorbent polymer material has a degree of neutralization of greater than 25%;
   wherein elements a) and b) are polymerized and prepared into superabsorbent polymer particles further comprising the following surface additives to form surface treated superabsorbent polymer particles:

   i) from 0.01 to 5% by weight of surface crosslinking agent based on the superabsorbent polymer material;
   ii) from 0.01 to 10% by weight of a penetration modifier based on the superabsorbent polymer material;
   iii) from 0 to 5% by weight of a multivalent metal salt based on the superabsorbent polymer material;
   iv) from 0 to 2% by weight of a surfactant based on the superabsorbent polymer material;
   v) from 0.01 to 5% by weight of an insoluble, inorganic powder based on the superabsorbent polymer material; and
   vi) from 0.01 to 5% by weight of a thermoplastic polymer based on the superabsorbent polymer material, said thermoplastic polymer comprising maleated polypropylene;

   wherein the surface treated superabsorbent polymer particles have been heat-treated to form the superabsorbent polymer material, and wherein the superabsorbent polymer particles further comprise a coating of from 0.01 to 5% by weight of a cationic polymer, based on the superabsorbent polymer material.

2. The absorbent article of claim 1 wherein the superabsorbent polymer material has a free swell gel bed permeability of at least 160 Darcy.

3. The absorbent article of claim 1 wherein the superabsorbent polymer material has a superabsorbent material shake-out of less than 16%.

4. The absorbent article of claim 1 wherein the superabsorbent polymer particles comprise from 0.1 to 5% by weight

of a multivalent metal salt.

5. The absorbent article of claim 1 wherein the thermoplastic polymer further comprises polyolefin, polyethylene, polyesters, polyurethanes, linear low density polyethylene (LLDPE), ethylene acrylic acid copolymer (EAA), styrene copolymers, ethylene alkyl methacrylate copolymer (EMA), polypropylene (PP), ethylene vinyl acetate copolymer (EVA), polyamide, polyester, or blends and copolymers thereof.

6. The absorbent article of claim 1 wherein the cationic polymer is a polyvinylamine.

7. The absorbent article of claim 1 wherein the superabsorbent polymer material has a gel bed permeability under 0.3 psi load of at least 4 Darcy, as measured by the Gel Bed Permeability Under Load Test.

8. The absorbent article of claim 1 wherein the superabsorbent polymer material has a centrifuge retention capacity of at least 25g/g.

9. The absorbent article of claim 1 wherein the insoluble, inorganic powder is silica.

10. The absorbent article of claim 1 wherein the absorbent core further comprises meltblown thermoplastic fiber.

11. The absorbent article of claim 1 wherein the absorbent core exhibits a composite permeability of at least 15 Darcy, as measured by the Composite Permeability Test.

12. The absorbent article of claim 1 wherein the absorbent core exhibits at least one of fluid intake rate value greater than 1 g/sec, as measured by the Fluid Intake Rate Test.

13. The absorbent article of claim 1 wherein the absorbent core exhibits a superabsorbent shakeout value not more than 2%, as measured by the Composite Shakeout Test.

14. The absorbent article of claim 1 wherein the superabsorbent polymer material comprises particles in a size range 300 to 600 microns.

15. The absorbent article of claim 1 wherein at least 50% of the particles are in a size range of 300 to 600 microns.

16. The absorbent article of claim 1 wherein the thermoplastic polymer is a blend of maleated polypropylene and ethylene acrylic acid copolymer.

17. The absorbent article of claim 1, wherein the absorbent core further comprises a meltblown thermoplastic elasteromeric fiber and a wood pulp fiber; and
wherein the absorbent core exhibits a stretchability of at least 50% as measured by the Composite Stretchability Test, a composite permeability of at least 15 Darcy as measured by the Composite Permeability Test, at least one fluid intake rate value greater than 1 g/sec as measured by the Fluid Intake Rate Test, and a superabsorbent shakeout value not more than 2% by weight as measured by the Composite Shakeout Test.

18. The absorbent article of claim 1 or 17 wherein the superabsorbent polymer material exhibits a centrifuge retention capacity of at least 23g/g as measured by the Centrifuge Retention Capacity Test, a free swell gel bed permeability of at least 100 Darcy as measured by the Free Swell Gel Bed Permeability Test, and a superabsorbent material shake-out of less than 20%, as measured by the Oven Shake-Out Test.

19. The absorbent article of claim 1 or 17 wherein at least one of the topsheet, backsheet, and absorbent core is stretchable.

20. The absorbent article of claim 1 or 17 wherein the absorbent core comprises layers.

21. The absorbent article of claim 1 or 17 wherein the absorbent core comprises three layers, and wherein the middle layer comprises an at least 10% by weight higher superabsorbent material percentage than the top layer and/or bottom layer of the absorbent core.

22. The absorbent article of claim 1 or 17 wherein the article is selected from personal care absorbent articles, health/medical absorbent articles, and household/industrial absorbent articles.

23. The absorbent article of claim 1 or 17 wherein the absorbent core comprises at least 60% by weight of the superabsorbent polymer material.

24. The absorbent article of claim 1 or 17 wherein the absorbent core comprises between 30% and 90% by weight of the superabsorbent materials.

25. The absorbent article of claim 1 or 17 wherein the absorbent core further comprises fluff.

26. The absorbent article of claim 1 or 17 wherein the absorbent core further comprises a surfactant.

27. The absorbent article of claim 10 or 17 wherein the absorbent core comprises between 5% to 35% by weight of the meltblown thermoplastic elastomeric fiber.

28. The absorbent article of claim 10 or 17 wherein the meltblown thermoplastic elastomeric fiber is stretchable.


**Patentansprüche**

1. Absorbierender Artikel, umfassend:

   eine Decklage;
   eine Außenlage; und
   einen zwischen der Decklage und der Außenlage angeordneten absorbierenden Kern;
   wobei der absorbierende Kern umfasst ein superabsorbierendes Polymermaterial,
   umfassend:

   a) von 55 bis 99,9 Gewichtsprozent, bezogen auf das superabsorbierende Polymer, polymerisierbares Monomer, welches eine ungesättigte Säuregruppe enthält, und
   b) von 0,001 bis 5 Gewichtsprozent, bezogen auf das polymerisierbare Monomer,
   welches eine ungesättigte Säuregruppe enthält, internes Vernetzungsmittel, wobei das superabsorbierende Polymermaterial einen Neutralisierungsgrad von mehr als 25% aufweist;

   wobei die Elemente a) und b) polymerisiert werden und zu superabsorbierenden Polymerpartikeln zubereitet werden, welche des Weiteren die nachfolgenden Oberflächenadditive umfassen, um oberflächenbehandelte superabsorbierende Polymerpartikel zu bilden:

   i) von 0,01 bis 5 Gewichtsprozent, bezogen auf das superabsorbierende Polymermaterial, Oberflächenvernetzungsmittel;
   ii) von 0,01 bis 10 Gewichtsprozent, bezogen auf das superabsorbierende Polymermaterial, eines Penetrationsmodifikators;
   iii) von 0 bis 5 Gewichtsprozent, bezogen auf das superabsorbierende Polymermaterial, eines mehrwertigen Metallsalzes;
   iv) von 0 bis 2 Gewichtsprozent, bezogen auf das superabsorbierende Polymermaterial, eines Detergens;
   v) von 0,01 bis 5 Gewichtsprozent, bezogen auf das superabsorbierende Polymermaterial, eines unlöslichen anorganischen Pulvers; und
   vi) von 0,01 bis 5 Gewichtsprozent, bezogen auf das superabsorbierende Polymermaterial, eines thermoplastischen Polymers, wobei das thermoplastische Polymer maleiniertes Polypropylen umfasst;

   wobei die oberflächenbehandelten superabsorbierenden Polymerpartikel wärmebehandelt worden sind, um das superabsorbierende Polymermaterial zu bilden, und wobei die superabsorbierenden Polymerpartikel des Weiteren einen Überzug von 0,01 bis 5 Gewichtsprozent, bezogen auf das superabsorbierende Polymermaterial, eines kationischen Polymers umfassen.

2. Absorbierender Artikel nach Anspruch 1, wobei das superabsorbierende Polymermaterial eine Gelbett-Permeabilität unter freien Quellbedingungen von mindestens 160 Darcy aufweist.

3. Absorbierender Artikel nach Anspruch 1, wobei das superabsorbierende Polymermaterial einen Superabsorbermaterial-Ausschüttelwert von weniger als 16% aufweist.

4. Absorbierender Artikel nach Anspruch 1, wobei die superabsorbierenden Polymerpartikel von 0,1 bis 5 Gewichtsprozent eines mehrwertigen Metallsalzes umfassen.

5. Absorbierender Artikel nach Anspruch 1, wobei das thermoplastische Polymer des Weiteren Polyolefin, Polyethylen, Polyester, Polyurethane, lineares Polyethylen niedriger Dichte (LLDPE), Ethylen-Acrylsäure-Copolymer (EAA), StyrolCopolymere, Ethylen-Alkylmethacrylat-Copolymer (EMA), Polypropylen (PP), Ethylen-Vinylacetat-Copolymer (EVA), Polyamid, Polyester, oder Gemische und Copolymere davon, umfasst.

6. Absorbierender Artikel nach Anspruch 1, wobei das kationische Polymer ein Polyvinylamin ist.

7. Absorbierender Artikel nach Anspruch 1, wobei das superabsorbierende Polymermaterial eine Gelbett-Permeabilität unter einer Last von 0,3 psi von mindestens 4 Darcy aufweist, wie mittels des Gelbett-Permeabilität-unter-Last-Tests gemessen.

8. Absorbierender Artikel nach Anspruch 1, wobei das superabsorbierende Polymermaterial eine Zentrifugenretentionskapazität von mindestens 25 g/g aufweist.

9. Absorbierender Artikel nach Anspruch 1, wobei das unlösliche anorganische Pulver Siliziumoxid ist.

10. Absorbierender Artikel nach Anspruch 1, wobei der absorbierende Kern des Weiteren schmelzgeblasene thermoplastische Faser umfasst.

11. Absorbierender Artikel nach Anspruch 1, wobei der absorbierende Kern eine Composit-Permeabilität von mindestens 15 Darcy aufweist, wie mittels des Composit-Permeabilität-Tests gemessen.

12. Absorbierender Artikel nach Anspruch 1, wobei der absorbierende Kern mindestens einen Fluidaufnahmegeschwindigkeit-Wert größer 1 g/s aufweist, wie mittels des Fluidaufnahmegeschwindigkeit-Tests gemessen.

13. Absorbierender Artikel nach Anspruch 1, wobei der absorbierende Kern einen Superabsorber-Ausschüttelwert von nicht mehr als 2% aufweist, wie mittels des Composit-Ausschüttel-Tests gemessen.

14. Absorbierender Artikel nach Anspruch 1, wobei das superabsorbierende Polymermaterial Partikel in einem Größenbereich von 300 bis 600 Mikrometern umfasst.

15. Absorbierender Artikel nach Anspruch 1, wobei mindestens 50% der Partikel in einem Größenbereich von 300 bis 600 Mikrometern liegen.

16. Absorbierender Artikel nach Anspruch 1, wobei das thermoplastische Polymer ein Gemisch von maleiniertem Polypropylen und Ethylen-Acrylsäure-Copolymer ist.

17. Absorbierender Artikel nach Anspruch 1, wobei der absorbierende Kern des Weiteren eine schmelzgeblasene thermoplastische elastomere Faser und eine Holzzellstofffaser umfasst; und
wobei der absorbierende Kern eine Dehnbarkeit von mindestens 50%, wie mittels des Composit-Dehnbarkeit-Tests gemessen, eine Composit-Permeabilität von mindestens 15 Darcy, wie mittels des Composit-Permeabilität-Tests gemessen, mindestens einen Fluidaufnahmegeschwindigkeit-Wert größer 1 g/s, wie mittels des Fluidaufnahmegeschwindigkeit-Tests gemessen, und einen Superabsorber-Ausschüttelwert von nicht mehr als 2%, wie mittels des Composit-Ausschüttel-Tests gemessen, aufweist.

18. Absorbierender Artikel nach Anspruch 1 oder 17, wobei das superabsorbierende Polymermaterial eine Zentrifugenretentionskapazität von mindestens 23 g/g, wie mittels des Zentrifugenretentionskapazität-Tests gemessen, eine Gelbett-Permeabilität unter freien Quellbedingungen von mindestens 100 Darcy, wie mittels des Gelbett-Permeabilität-unter-freien-Quellbedingungen-Tests gemessen, einen Superabsorbermaterial-Ausschüttelwert von weniger als 20%, wie mittels des Ofen-Ausschüttel-Tests gemessen, aufweist.

19. Absorbierender Artikel nach Anspruch 1 oder 17, wobei mindestens eine Komponente aus Decklage, Außenlage und absorbierendem Kern dehnbar ist.

**20.** Absorbierender Artikel nach Anspruch 1 oder 17, wobei der absorbierende Kern Schichten umfasst.

**21.** Absorbierender Artikel nach Anspruch 1 oder 17, wobei der absorbierende Kern drei Schichten umfasst, und wobei die mittlere Schicht einen mindestens 10 Gewichtsprozent höheren Prozentanteil an superabsorbierendem Material umfasst als die obere Schicht und/oder die untere Schicht des absorbierenden Kerns.

**22.** Absorbierender Artikel nach Anspruch 1 oder 17, wobei der Artikel ausgewählt ist aus absorbierenden Körperhygieneartikeln, absorbierenden Gesundheitsvorsorgeartikeln/medizinischen Artikeln und absorbierenden Haushaltsartikeln/Industrieartikeln.

**23.** Absorbierender Artikel nach Anspruch 1 oder 17, wobei der absorbierende Kern mindestens 60 Gewichtsprozent des superabsorbierenden Polymermaterials umfasst.

**24.** Absorbierender Artikel nach Anspruch 1 oder 17, wobei der absorbierende Kern zwischen 30 Gewichtsprozent und 90 Gewichtsprozent der superabsorbierenden Materialien umfasst.

**25.** Absorbierender Artikel nach Anspruch 1 oder 17, wobei der absorbierende Kern des Weiteren Fluffzellstoff umfasst.

**26.** Absorbierender Artikel nach Anspruch 1 oder 17, wobei der absorbierende Kern des Weiteren ein Detergens umfasst.

**27.** Absorbierender Artikel nach Anspruch 10 oder 17, wobei der absorbierende Kern zwischen 5 Gewichtsprozent und 35 Gewichtsprozent der schmelzgeblasenen thermoplastischen elastomeren Faser umfasst.

**28.** Absorbierender Artikel nach Anspruch 10 oder 17, wobei die schmelzgeblasene thermoplastische elastomere Faser dehnbar ist.

**Revendications**

**1.** Article absorbant comprenant :

une feuille supérieure ;
une feuille dossier ; et
un noyau absorbant disposé entre la feuille supérieure et la feuille dossier ;
article dans lequel le noyau absorbant comprend un matériau polymère superabsorbant comprenant :

a) de 55 à 99,9 % en poids, sur la base du polymère superabsorbant, d'un monomère polymérisable contenant un(des) groupe(s) acide(s) insaturé(s), et
b) de 0,001 à 5 % en poids, sur la base du monomère polymérisable contenant un (des) groupe (s) acide (s) insaturé(s), d'un agent de réticulation interne ;

le matériau polymère superabsorbant ayant un degré de neutralisation supérieur à 25 % ;
les éléments a) et b) étant polymérisés et transformés en particules de polymère superabsorbant comprenant, en outre, les additifs de surface suivants pour former des particules de polymère superabsorbant traitées en surface :

i) de 0,01 à 5 % en poids, sur la base du matériau polymère superabsorbant, d'un agent de réticulation superficielle ;
ii) de 0,01 à 10 % en poids, sur la base du matériau polymère superabsorbant, d'un modificateur de pénétration ;
iii) de 0 à 5 % en poids, sur la base du matériau polymère superabsorbant, d'un sel métallique multivalent ;
iv) de 0 à 2 % en poids, sur la base du matériau polymère superabsorbant, d'un tensioactif ;
v) de 0,01 à 5 % en poids, sur la base du matériau polymère superabsorbant, d'une poudre inorganique insoluble ; et
vi) de 0,01 à 5 % en poids, sur la base du matériau polymère superabsorbant, d'un polymère thermoplastique, ledit polymère thermoplastique comprenant du polypropylène maléaté ;

les particules de polymère superabsorbant traitées en surface ayant été traitées thermiquement pour former le matériau polymère superabsorbant et les particules de polymère superabsorbant comprenant, en outre, un enrobage formé de 0,01 à 5 % en poids, sur la base du matériau polymère superabsorbant, d'un polymère cationique.

2. Article absorbant selon la revendication 1, dans lequel le matériau polymère superabsorbant a une perméabilité sur lit de gel gonflant librement d'au moins 160 Darcy.

3. Article absorbant selon la revendication 1, dans lequel le matériau polymère superabsorbant a une perte de matériau superabsorbant au secouage inférieure à 16 %.

4. Article absorbant selon la revendication 1, dans lequel les particules de polymère superabsorbant comprennent de 0,1 à 5 % en poids d'un sel métallique multivalent.

5. Article absorbant selon la revendication 1, dans lequel le polymère thermoplastique comprend, en outre, une polyoléfine, un polyéthylène, des polyesters, des polyuréthanes, un polyéthylène linéaire basse densité (LLDPE), un copolymère éthylène-acide acrylique (EAA), des copolymères de styrène, un copolymère d'éthylène et de méthacrylate d'alkyle (EMA), un polypropylène (PP), un copolymère éthylène-acétate de vinyle (EVA), un polyamide, un polyester, ou des mélanges et copolymères de ceux-ci.

6. Article absorbant selon la revendication 1, dans lequel le polymère cationique est une polyvinylamine.

7. Article absorbant selon la revendication 1, dans lequel le matériau polymère superabsorbant a une perméabilité sur lit de gel, sous une charge de 0,3 livre/pouce$^2$, d'au moins 4 Darcy, telle que mesurée par le Test de Perméabilité sur Lit de Gel sous Charge.

8. Article absorbant selon la revendication 1, dans lequel le matériau polymère superabsorbant a une capacité de rétention centrifuge d'au moins 25 g/g.

9. Article absorbant selon la revendication 1, dans lequel la poudre inorganique insoluble est de la silice.

10. Article absorbant selon la revendication 1, dans lequel le noyau absorbant comprend, en outre, des fibres thermoplastiques obtenues par extrusion-soufflage.

11. Article absorbant selon la revendication 1, dans lequel le noyau absorbant manifeste une perméabilité du composite d'au moins 15 Darcy, telle que mesurée par le Test de Perméabilité du Composite.

12. Article absorbant selon la revendication 1, dans lequel le noyau absorbant manifeste au moins une valeur de vitesse de capture de fluide supérieure à 1 g/s, telle que mesurée par le Test de Vitesse de Capture de Fluide.

13. Article absorbant selon la revendication 1, dans lequel le noyau absorbant manifeste une valeur de perte de matériau superabsorbant au secouage n'excédant pas 2 %, telle que mesurée par le Test de Perte au Secouage du Composite.

14. Article absorbant selon la revendication 1, dans lequel le matériau polymère superabsorbant comprend des particules ayant une granulométrie comprise entre 300 et 600 microns.

15. Article absorbant selon la revendication 1, dans lequel au moins 50 % des particules ont une granulométrie comprise entre 300 et 600 microns.

16. Article absorbant selon la revendication 1, dans lequel le polymère thermoplastique est un mélange de polypropylène maléaté et de copolymère éthylène-acide acrylique.

17. Article absorbant selon la revendication 1, dans lequel le noyau absorbant comprend, en outre, des fibres élastomères thermoplastiques obtenues par extrusion-soufflage et des fibres de pâte de bois ; et
le noyau absorbant manifeste une extensibilité d'au moins 50 % telle que mesurée par le Test d'Extensibilité du Composite, une perméabilité du composite d'au moins 15 Darcy telle que mesurée par le Test de Perméabilité du Composite, au moins une valeur de vitesse de capture de fluide supérieure à 1 g/s telle que mesurée par le Test de Vitesse de Capture de Fluide et une valeur de perte de matériau superabsorbant au secouage n'excédant pas 2 % en poids, telle que mesurée par le Test de Perte au Secouage du Composite.

18. Article absorbant selon la revendication 1 ou 17, dans lequel le matériau polymère superabsorbant manifeste une capacité de rétention centrifuge d'au moins 23 g/g telle que mesurée par le Test de Capacité de Rétention Centrifuge, une perméabilité sur lit de gel gonflant librement d'au moins 100 Darcy telle que mesurée par le Test de Perméabilité sur Lit de Gel Gonflant Librement et une valeur de perte de matériau superabsorbant au secouage inférieure à 20 % en poids, telle que mesurée par le Test de Perte au Secouage dans un Four.

19. Article absorbant selon la revendication 1 ou 17, dans lequel l'un au moins de la feuille supérieure, de la feuille dossier et du noyau absorbant est extensible.

20. Article absorbant selon la revendication 1 ou 17, dans lequel le noyau absorbant comprend des couches.

21. Article absorbant selon la revendication 1 ou 17, dans lequel le noyau absorbant comprend trois couches et dans lequel la couche médiane comprend une concentration de matériau superabsorbant supérieure d'au moins 10 % en poids à celle de la couche supérieure et/ou de la couche inférieure du noyau absorbant.

22. Article absorbant selon la revendication 1 ou 17, ledit article étant sélectionné parmi les articles absorbants d'hygiène personnelle, les articles absorbants sanitaires/médicaux, et les articles absorbants domestiques/industriels.

23. Article absorbant selon la revendication 1 ou 17, dans lequel le noyau absorbant comprend au moins 60 % en poids du matériau polymère superabsorbant.

24. Article absorbant selon la revendication 1 ou 17, dans lequel le noyau absorbant comprend de 30 % à 90 % en poids de matériaux superabsorbants.

25. Article absorbant selon la revendication 1 ou 17, dans lequel le noyau absorbant comprend, en outre, du duvet.

26. Article absorbant selon la revendication 1 ou 17, dans lequel le noyau absorbant comprend, en outre, un tensioactif.

27. Article absorbant selon la revendication 10 ou 17, dans lequel le noyau absorbant comprend entre 5 % et 35 % en poids de fibres élastomères thermoplastiques obtenues par extrusion-soufflage.

28. Article absorbant selon la revendication 10 ou 17, dans lequel les fibres élastomères thermoplastiques obtenues par extrusion-soufflage sont extensibles.

FIG. 1

FIG. 2

FIG. 3

EP 1 960 007 B1

EP 1 960 007 B1

FIG. 4

50

55　　　　　　　　　53　　　　　55

54　　　　　　　　　　　　　　　　54

51

52　　　　　　58

# FIG. 5A

50

54

51

59

52

53

58

54

# FIG. 5B

FIG. 6

FIG. 7

FIG. 8

FIG. 9

FIG. 10

FIG. 11

EP 1 960 007 B1

400

402

418

412

5

408

416

414

5

**FIG. 12**

412

402

416 407 410 409 408 416

414 414

418

406

404

**FIG. 13**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0037009 A, A. Fletcher
- US 4940464 A, Van Gompel
- US 5766389 A, Brandon
- US 6645190 A, Olson
- US 5883028 A, Morman
- US 5116662 A, Morman
- US 5114781 A, Morman
- US 6552245 B, Roessler
- US 6641134 B, Vukos
- US 5486166 A, Bishop
- US 5490846 A, Ellis
- US 5820973 A, Dodge
- US 3849241 A
- US 5350624 A
- US 4100324 A, Anderson
- US 4587154 A, Hotchkiss
- US 4604313 A, McFarland
- US 4655757 A, McFarland
- US 4724114 A, McFarland
- GB 2151272 A, Minto
- US 6362389 B, D. J. McDowall
- US 883174 A, X. Zhang
- US 6090875 A
- US 5994440 A
- WO 02076520 A
- US 20020183703 A1

### Non-patent literature cited in the description

- Surface and Colloid Science - Experimental Methods. Plenum Press, 1979, vol. II